# EUROPÄISCHE PATENTSCHRIFT

(11) **EP 1 675 598 B1**
(45) Veröffentlichungstag und Bekanntmachung des Hinweises auf die Patenterteilung: **16.09.2009**
(21) Anmeldenummer: 04790826.4
(22) Anmeldetag: 25.10.2004
(51) Int. Cl.: A23L 1/304, A61K 33/04, A61K 33/16, A61K 33/18, A61K 33/24, A61K 33/26, A61K 33/30

(54) **SPURENELEMENTZUSAMMENSETZUNG FÜR DIE ERNÄHRUNG**
NUTRITION TRACE ELEMENT COMPOSITION
COMPOSITION D'OLIGO-ELEMENTS DESTINES A L'ALIMENTATION

(30) Priorität: 24.10.2003 DE 10349585
(43) Veröffentlichungstag der Anmeldung: 05.07.2006
(73) Patentinhaber: Biosyn Arzneimittel GmbH, 70734 Fellbach (DE)
(72) Erfinder: STIEFEL, Thomas, 70184 Stuttgart (DE)
(74) Vertreter: Grünecker, Kinkeldey, Stockmair & Schwanhäusser Anwaltssozietät
(86) Internationale Anmeldenummer: PCT/EP2004/012040
(87) Internationale Veröffentlichungsnummer: WO 2005/039604

(56) Entgegenhaltungen:
- US-A1- 2003 161 863
- US-B1- 6 391 332
- FRANKEL, D. A.: "Supplementation of trace elements in parenteral nutrition: rationale and recommendations" NUTRITION RESEARCH, Bd. 13, 1993, Seiten 583-596, XP008040636

## Beschreibung

Die vorliegende Erfindung betrifft eine Zusammensetzung mit Spurenelementen (Spurenelementzusammensetzung) für die Ernährung.

Kommerziell versteht man unter solchen "Spurenelementzusammensetzungen für die Ernährung" Elektrolytkonzentrate. Sie werden z.B. als Zusatz zu Infusionslösungen in der Roten Liste geführt.

In der vorliegenden Erfindung soll der Begriff als dementsprechend definiert gelten, d.h. "Spurenelementzusammensetzungen für die Ernährung", enthaltend ausschließlich Elektrolytkonzentrate.

Die parenterale Ernährung ist eine spezielle Form der Nährstoff und Flüssigkeitszufuhr. Sie unterscheidet sich von der normalen oralen Nahrungsaufnahme dadurch, dass die Stoffe über einen künstlich geschaffenen venösen Zugang mittels Infusion in den Organismus gelangen. Der gesamte Verdauungstrakt wird dadurch umgangen. Die Indikation zur intravenösen Nährstoffversorgung ist immer dann gegeben, wenn die Aufnahme oral zugeführter Nahrung über den Verdauungsweg nicht möglich, nicht erwünscht oder zu gefährlich ist. Im Allgemeinen kommt die parenteralen Ernährung bei erheblichen Störungen der Verdauung und Resorption sowie im Rahmen der Intensivmedizin zur Anwendung. Eine vollständig parenterale Ernährung sollte die gleichen Nährstoffe liefern, wie die normale enterale Nahrung: Kohlenhydrate, Fette, Proteine, Vitamine, Elektrolyte, Wasser und auch Spurenelemente.

Eine künstliche Ernährung kranker Menschen konnte lange Zeit nur auf oralem Weg erfolgen. Im 16. Jahrhundert soll erstmals die Sondentechnik eingesetzt worden sein. Die Grundlage zur Infusionstherapie erbrachten die Erkenntnisse über den Körperkreislauf, den der britische Arzt W. Harvey 1628 beschrieb. In den kommenden Jahrhunderten kam die Infusionstechnik nur langsam voran. Versuche, beispielsweise Cholera-Patienten Milch zu infundieren, schlugen fehl. Durch die Entwicklung der Sterilisation und geeigneter Infusionslösungen gelang der parenteralen Ernährung der Durchbruch. Ende des 19. Jahrhunderts kamen Kochsalz- und Zuckerlösungen zum Einsatz Die erste A-minosäureinfusion erfolgte 1937 durch Elman. Erst im Jahr 1961 gelang es, eine sichere und verwertbare Fettemulsion für den klinischen Gebrauch zu entwickeln. Die Gabe von Mengen- und besonders Spurenelementen wurde lange nicht berücksichtigt Spurenelemente sind für die Aufrechterhaltung körperlicher und geistiger Leistungsfähigkeit von Bedeutung. Als strukturelle bzw. funktionelle Bestandteile zahlreicher Metalloproteine (Kupfer, Zink), Enzyme (Selen), Hormone (Iod) oder Vitamine (Kobalt) sind Spurenelemente an vielen Stoffwechselprozessen beteiligt.

Vor der Einführung der intravenösen Nährstoffversorgung in die klinische Medizin betrafen Spurenelementdefizite ausschließlich Bevölkerungsgruppen in geologischen Mangelgebieten. Klinische Mangelzustände traten in Europa und Amerika aufgrund von geochemischen und emährungsbedingten Umständen bis auf den klassischen lodmangel so gut wie nie auf. Erst durch die parenterale Ernährung kam es in den westlichen Ländern zu isolierten Mangelerscheinungen als Folge einer ungenügenden Spurenelementzufuhr.

Ein Mangel an Spurenelementen beeinträchtigt das optimale Ablaufen wichtiger physiologischer Vorgänge im Körper. Der Spurenelementmangel entwickelt sich unter parenteraler Ernährung in mehreren Stadien. Bei unzureichender Zufuhr greift der Organismus auf endogene Spurenelementspeicher zurück. Durch die Entleerung dieser Speicher macht sich der Mangel zunächst durch unspezifische Anzeichen bemerkbar, die sich über spezifische Stoffwechselstörungen bis hin zu den für jedes Spurenelement charakteristischen Mangelsymptomen entwickeln. Die klassischen Mangelsymptome sind in frühen Stadien reversibel. Bei fehlender Substitution können sie jedoch in irreversible metabolische Funktionsstörungen übergehen, die in einem späteren Stadium sogar potenziell letal sein können.

Eine optimale Spurenelementsupplementierung stellt somit eine wichtige Komponente im Rahmen der künstlichen Ernährungstherapie dar. Im Vordergrund steht dabei die Verhinderung von metabolischen Funktionsstörungen, die auf die Infusionstherapie selbst zurückzuführen sind. Ein weiteres Ziel einer adäquaten Spurenelementversorgung ist es, einen bereits vorhandenen Mangel zu beheben und dem Patienten zu einer höheren Lebensqualität zu verhelfen.

Die durch einen Spurenelementmangel hervorgerufenen Funktionsstörungen werden üblicherweise anhand von erniedrigten Spurenelementkonzentrationen im Blut bestimmt, aus denen sich jedoch der ernährungsphysiologische Spurenelementstatus nur bedingt ableiten läßt. Neuere Empfehlungen orientieren sich zunehmend an Aktivitätsverlusten von Spurenelementabhängigen Enzymen bzw. an veränderten Stoffwechselprozessen, an denen Spurenelemente beteiligt sind. Hierzu fehlen noch weitgehend die nötigen diagnostischen Methoden. Bedarfsbestimmungen, die sich an biochemischen Parametern orientieren, liegen bislang nur für einige Spurenelemente vor. Auch analytische Verfahren für einige seltene Spurenelemente sind nicht an allen Kliniken verfügbar. Somit wird auch weiterhin zur Bestimmung des Spurenelementstatus der Blutspiegel der einzelnen Elemente als Orientierung genutzt (Gramm et al. 1992).

Welche Spurenelemente für den Menschen als essenziell angesehen werden, hängt vom aktuellen wissenschaftlichen Erkenntnisstand ab. Heute gelten die Spurenelemente Chrom, Eisen, Fluor, Iod, Kupfer, Mangan, Molybdän, Selen, sowie Zink als essenziell, daneben auch Kobalt als Bestandteil von Vitamin B₁₂. In letzter Zeit wird diskutiert, dass auch weitere Spurenelemente als essenziell eingestuft werden können - Zinn, Silizium, Vanadium, Arsen, Blei, Aluminium, Lithium, Cadmium, Bor. Die biologischen Funktionen dieser Spurenelemente und vor allem ihre Essentialität für den Menschen ist jedoch noch nicht vollständig erforscht, so dass über den Bedarf noch keine zuverlässigen Angaben gemacht werden können. Die toxische Wirkung dieser Elemente steht noch immer im Vordergrund.

Eine sinnvolle Spurenelementzufuhr setzt die Kenntnis über den intravenösen Bedarf an den essentiellen Spurenelementen voraus. Während der Tagesbedarf des Gesunden unter Berücksichtigung seiner individuellen Lebensumstände (Schwangerschaft, Wachstumsperiode, Leistungssportler) weitgehend bekannt ist, ist es schwierig, eine Empfehlung für den Kranken zu geben. Dafür gibt es mehrere Gründe.
1. Die Empfehlungen für die parenterale Zufuhr von Spurenelementen orientieren sich an den Empfehlungen für die orale Zufuhr und werden um die jeweilige intestinale Resorptionsrate korrigiert. Aber selbst die Werte für die orale Zufuhr stehen zum Teil auf nur schwachem Fundament, da brauchbare langfristige Bilanzuntersuchungen mit allgemein anerkannten analytischen Methoden nur in Einzelfällen vorliegen.
2. Die benötigte Menge an Spurenelementen ist stets vom Individuum selbst und der biologischen Situation (Krankheit, Streß) abhängig und steht dabei immer in Relation zum Stoffwechsel. Da die Hauptfunktion der Spurenelemente darin besteht, dass sie als Cofaktoren oder prosthetische Gruppen einer Reihe von Enzymen fungieren, schwankt der Bedarf in Abhängigkeit von den Veränderungen des Enzym- und Proteinmusters und der metabolischen Aktivität während einer Krankheit. Mit zunehmendem Energieumsatz steigt der Spurenelementumsatz an, z.B. der Zinkbedarf bei Kohlenhydratzufuhr.
3. Mit Verlusten in Sekreten, Drainageflüssigkeiten und bei Diarrhö ist bei einigen Patienten auch zu rechnen, deren Höhe aber nicht genau bekannt ist.
4. Zudem sind Resorptionsquoten für die einzelnen Elemente nur schätzungsweise bekannt. Sie können zwischen 1 und 90 % liegen und sind angebots- und stoffwechselabhängig. Die Kenntnis dieser Daten ist aber Voraussetzung für die Übertragung der oralen Empfehlungen auf die parenterale Zufuhr.
5. Spezielle Untersuchungen über den Spurenelementbedarf während parenteraler Ernährung oder bei verschiedenen Krankheitszuständen existieren so gut wie nicht.

In der folgenden Tabelle sind die neun essentiellen Spurenelemente, ihre Funktionen, die möglichen Mangelsymptome sowie die oralen Zufuhrempfehlungen der Deutschen Gesellschaft für Ernährung (DGE) für den gesunden Erwachsenen angegeben (DGE2000).

**Tabelle 1 Essentielle Spurenelemente**

| **Element** | **Funktion** | **Mangelsymptome** | **orale empfohlene Zufuhr** |
|---|---|---|---|
| Chrom | Beteiligung am Kohlenhydratstoffwechsel, Verbesserung der Glukosetoleranz | gestörte Glukoseverwertung, Gewichtsverlust, Enzephalopathie | 30 - 100 µg/d |
| Eisen | Bestandteil sauerstoffübertragender Wirkgruppen (Hämoglobin, Myoglobin) | Eisenmangelanämie, erhöhte Infektionsanfälligkeit, Beeinträchtigung der körperlichen Leistungsfähigkeit | 10 - 15 mg/d |
| Fluor | Knochen- und Zahnaufbau, Kariesprophylaxe | Karies | 3,1 - 3,8 mg/d |
| Iod | Bestandteil der Schilddrüsenhormone | Hypothyreose, Struma | 180 - 200 µg/d |
| Kupfer | Bestandteil von Enzymen, die an Redoxprozessen beteiligt sind, wichtig im Eisenstoffwechsel, beteiligt an der Synthese von Kollagen und Elastin | hypochrome mikrozytäre Anämie, Leukozytopenie, Granulozytopenie, Knochenfrakturen, Gefäßrupturen, Aneurysmen, neurologische Störungen | 1,0-1,5mg/d |
| Mangan | Bestandteil zahlreicher Metalloenzyme, Aktivierung anderer Enzyme | beim Menschen nicht beobachtet, bei anderen Spezies: Wachstums- 2,0-störungen, gestörter Fett- und Kohlenhydrat-stoffwechsel | 5,0 mg/d |
| Molybdän | Bestandteil mehrerer Enzyme | beim Menschen nicht bekannt | 50 - 100µg/d |
| Selen | Antioxidative Wirkung, Inaktivierung von freien Radikalen, Schutz der Membranen, auch antikanzerogene und immunstärkende Wirkung | Kardiomyopathie, Myopathie der Skelettmuskulatur, Atrophie der Muskelfasern, erniedrigte T-Lymphozytenaktivität, erythrozytäre Makrozytose, Hämolysenelgung | 30 - 70 µgd |
| Zink | Bestandteil zahlreicher Enzyme, wichtig für die Wundheilung, im Nukleinstoffwechsel, für die Spelcherung von Insulin, beteillgt am Transport von Kohlendioxid im Blut | Wachstumsstillstand, Gewichtsverlust, gestört Wundhellung, therapieresistente Diarrböen, Dermatitis, Depression der zellulären Immunantwort, erhöhte Infektionsanfälligkelt, neurologische Erscheinungen | 7 - 10 mg/d |

Bei parenteraler Ernährung sollten nach heutigern Wissen die Spurenelemente Chrom, Eisen, Fluor, Iod, Kupfer, Mangan, Molybdän, Selen sowie Zink substituiert werden. Bei dem Präparat Tracutil^{®} handelt es sich um eine Spurenelementlösung für die parenterale Ernährung der B. Braun Melsungen AG. Die o.g. Spurenelemente sind in Tracutil^{®} enthalten.

Heute gelten die Spurenelemente Chrom, Eisen, Fluor, Iod, Kupfer, Mangan, Molybdän, Selen, sowie Zink als essentiell, daneben auch Kobalt als Bestandteil von Vitamin B₁₂. Diese Spurenelemente sollten auch während einer parenteralen Ernährung zugeführt werden.

Durch die Präparate Inzolen HK und Inzolen^{®} sine NaCL wurden neben den Mengenelementen Chlor, Kalium, Magnesium und Natrium die Spurenelemente Kobalt, Kupfer, Mangan und Zink verabreicht.

Der Bedarf an Selen sollte durch die Gabe von selenase^{®} und ein erhöhter Bedarf an Zink durch die Verabreichung von Unizink^{®} gedeckt werden.

Die kombinierte Zufuhr von Mengen- und Spurenelementen in einer Lösung - wie in diesem Fall mit den Präparaten Inzolen HK, Inzolen^{®} sine NaCL - und die Ausrichtung der Supplementierung am Kallum-Wert, erwies sich als ungünstig. Weiterhin konnte der sporadische Einsatz von selenase^{®} und Unizink^{®} eine optimale Versorgung der Patienten mit Selen und Zink nicht gewährleisten.

FRANKEL, D. A.: "Supplementation of trace elements in parenteral nutrition: rationale and recommendations" NUTRITION RESEARCH, Bd. 13, 1993, Seiten 583-596 offenbart Empfehlungen für die Spurenelementversorgung, wobei Se mit einer Maximaldosis von 250 µg pro Tag angegeben wird.

Weitere kommerzielle Spurenelementpräparate sind "Decan®" und "Mikro + Pediatric^{™}", die jedoch verschiedene Nachteile, wie in Tabelle 2 und 3 illustriert, aufweisen.

**Tabelle 2**

| | | Micro+6 PediatricTM | | Empfehlungen, parenterale Zufuhr/Tag American Medical Association 1979, 1984; Fleming 1989; Berger 1995, Shenkin 1995 | | | |
|---|---|---|---|---|---|---|---|
| **Fe²⁺** | 55,8 | 0,00 mg | 0,00 µmol | 1,2 | mg | 21,49 | µmol |
| | | | | | | | |
| **Zn²⁺** | 65,4 | 3,00 mg | 45,9 µmol | 2,4-15 2,5-5 | mg | 36,70 | µmol |
| | | | | | | 229,39 | |
| | | | | | | | |
| **Mn²⁺** | 54,9 | 0,10 mg | 1,82 µmol | 0,15-0,8 0,06-0,1 | mg | 2,73 14,56 | µmol |
| | | | | | | | |
| **Cu²⁺** | 63,6 | 0,40 mg | 6,29 µmol | 0,3-1,6 0,3-0,5 | mg | 4,72 25,18 | µmol |
| | | | | | | | |
| **F⁻** | 19 | 0,00 mg | 0,00 µmol | 0,95 | mg | 50,03 | µmol |
| | | | | | | | |
| **J⁻** | 127 | 0,06 mg | 0,47 µmol | 0,131 | mg | 1,03 | µmol |
| | | | | | | | |
| **Cr³⁺** | 52 | 0,04 mg | 0,77 µmol | 0,01-0,03 0,01-0,015 | mg | 0,190,58 | µmol |
| | | | | | | | |
| **Se⁴⁺** | 79 | 0,02 mg | 0,25 µmol | 0,03-0,5 | mg | 0,38 6,33 | µmol |
| | | | | | | | |
| **Mo⁴⁺** | 95,9 | 0,00 mg | 0,00 µmol | 0,019-0,2 | mg | 0,20 2,08 | µmol |

| | | | | | | | |
|---|---|---|---|---|---|---|---|
| **Fe²⁺:** "Null" entspricht zwar nicht den Empfehlungen; neuere Untersuchungen (u.a. Mette Berger 2004; ESICM Berlin) zeigen jedoch, dass eine Eisenzufuhr keine Vorteile hat, eher zur Radikalbelastung beiträgt. **Zn²⁺:** 3 mg entsprechen den bisherigen Zufuhrempfehlungen; verschiedene Untersuchungen deuten jedoch darauf hin, dass bei einem vorliegenden Zinkmangel, um diesen zu beheben, 30-40 mg täglich zugeführt werden müssen (z.B. Hackl 1992) **Mn²⁺:** 0,1 mg entsprechen den Mindestempfehlungen. Erhöhte Mangankonzentrationen sind laut Literaturangaben als gefährlich anzusehen. Die hauptsächliche Komplikation ist eine Ablagerung von Mangan in den Basalganglien, die mit Parkinson-ähnlichen Symptomen verbunden sein kann und wahrscheinlich nicht reversibel ist. Die Manganspiegel sollten deshalb regelmäßig bestimmt werden (Shenkin 2001). **Cu²⁺:** 0,4 mg entsprechen den Mindestempfehlungen. Aus der Literatur ist bekannt, dass die Kupfermengen aller Blutfraktionen miteinander in Verbindung stehen und deren Kupfergehalte nicht (direkt) von der alimentären Zufuhr ab-hängen, sondern von Faktoren wie Tagesrhythmus, Geschlecht, Alter, Hormonstatus, inflammatori-schen Prozessen u.v.m.. Es findet also ein ständiger Austausch von zwischen Intra- und Extrazellulär-raum statt (Rükgauer und Kruse-Jarres 2000). **F⁻:** "Null" entspricht nicht den Empfehlungen; neuere Untersuchungen (biosyn) zeigen jedoch, dass über fluorhaltige Anästhetika meist eher zuviel Fluor zugeführt wird, so dass für eine kurzfristige parenterale Ernährung keine zusätzliche F-Gabe notwendig wäre; allerdings wird angegeben, dass physiologisch wirksam nur die anorganischen Fluor-Verbindungen sind (Heseker 1999) und die organischen so nicht als Fluorzufuhr gewertet werden können. **J⁻**:0,06 mg entsprechen ca. der Hälfte der Zufuhrempfehlungen; da die lodversorgung der Patienten durch iodhaltige Kontrast- und Desinfektionsmittel meist übernormal ist, dürfte es es so nicht zu einem lodmangel kommen; es stellt sich allerdings die Frage, ob die Zufuhr von Iod auf diesem Wege überhaupt zu empfehlen ist: Bei hoher lodzufuhr besteht die Gefahr eines akuten Anstiegs der Schilddrüsenhormone bei Patienten mit vorausgehender lodmangelernährung. (Eine normal funktionierende Schilddrüse mit ausreichender lodversorgung ist in der Lage, die Absorption und Bildung organischer lodverbindungen zu hemmen (Wolff-Chaikoff-Effekt)). **Cr³⁺:** 0,04 mg sind etwas mehr als die Empfehlungen; möglicherweise wird aber den neueren Erkenntnissen Rechnung getragen (Berger 2004), dass eine Zufuhr von 0,04 mg die Glykämie sowie Insulinämie reduziert. In anderen Untersuchungen (biosyn) wurde allerdings festgestellt, dass es in der Regel nicht zur Cr-Unterversorgung kommt, da viele Lösungen mit Cr kontaminiert sind (Shenkin 2001). **Se⁴⁺:** 0,02 mg liegen sogar unter den Zufuhrempfehlungen; zahlreiche Untersuchungen zeigen aber (z.B. biosyn), dass eine tägliche Gabe von 1 mg von Vorteil sind und nicht zu übernormalen Blutspiegeln führen. **Mo⁴⁺:** "Null" entspricht nicht den Zufuhrempfehlungen; zu Mo treten aber in der Literatur oft widersprüchliche Daten auf, die sich möglicherweise auf Messschwierigkeiten zurückführen lassen; z B. können hohe Werte schon durch Kontamination bei der Blutabnahme mit Stahlkanülen erzeugt werden (Versieck 1983). | | | | | | | |

**Tabelle 3**

| | | Baxter Decan® | | | | Empfehlungen, parenterale Zufuhr/Tag American Medical Association 1979, 1984; Fleming 1989; Berger 1995, Shenkin 1995 | | | |
|---|---|---|---|---|---|---|---|---|---|
| **Fe²⁺** | 55,8 | 1,00 | mg | 17,90 | µmol | 1,2 | mg | 21,49 | µmol |
| | | | | | | | | | |
| **Zn²⁺** | 65,4 | 10,00 | mg | 153,00 | µmol | 2,4-15 2,5-5 | mg | 36,70 229,39 | µmol |
| | | | | | | | | | |
| **Mn²⁺** | 54,9 | 0,20 | mg | 3,64 | µmol | 0,15-0,8 0,06-0,1 | mg | 2,73 14,56 | µmol |
| | | | | | | | | | |
| **Cu²⁺** | 63,6 | 0,48 | mg | 7,55 | µmol | 0,3-1,6 0,3-0,5 | mg | 4,72 25,18 | µmol |
| | | | | | | | | | |
| **F⁻** | 19 | 1,45 | mg | 76,30 | µmol | 0,95 | mg | 50,03 | µmol |
| | | | | | | | | | |
| **J⁻** | 127 | 0,0015 | mg | 0,012 | µmol | 0,131 | mg | 1,03 | µmol |
| **Cr³⁺** | 52 | 0,015 | mg | 0,289 | µmol | 0,01-0,03 0,01-0,015 | mg | 0,19 0,58 | µmol |
| | | | | | | | | | |
| **Se⁴⁺** | 79 | 0,07 | mg | 0,887 | µmol | 0,03-0,5 | mg | 0,38 6,33 | µmol |
| | | | | | | | | | |
| **Mo⁴⁺** | 95,9 | 0,025 | mg | 0,261 | µmol | 0,019-0,2 | mg | 0,20 2,08 | µmol |
| **Co** | 58,9 | 0,074 | mg | 1,261 | µmol | - | | - | - |

| | | | | | | | | | |
|---|---|---|---|---|---|---|---|---|---|
| **Fe²⁺:** 1 mg liegt leicht unter den Empfehlungen; neuere Untersuchungen (u.a. Mette Berger 2004; ESICM Berlin) zeigen jedoch, dass eine Eisenzufuhr keine Vorteile hat, eher zur Radikalbelastung beiträgt. **Zn²⁺:** 10 mg entsprechen den bisherigen Zufuhrempfehlungen, liegen aber im Vgl. zu anderen Lösungen schon sehr hoch; verschiedene Untersuchungen deuten darauf hin, dass bei einem vorliegenden Zinkmangel, um diesen zu beheben, 30-40 mg täglich zugeführt werden müssen (z.B. Hackl 1992) **Mn²⁺:** 0,2 mg entsprechen den Empfehlungen. Erhöhte Mangankonzentrationen sind laut Literaturangaben als gefährlich anzusehen. Die hauptsächliche Komplikation ist eine Ablagerung von Mangan in den Basalganglien, die mit Parkinson-ähnlichen Symptomen verbunden sein kann und wahrscheinlich nicht reversibel ist. Die Manganspiegel sollten deshalb regelmäßig bestimmt werden (Shenkin 2001). **Cu²⁺:** 0,48 mg entsprechen den Empfehlungen. Aus der Literatur ist bekannt, dass die Kupfermengen aller Blutfraktionen miteinander in Verbindung stehen und deren Kupfergehalte nicht (direkt) von der alimentären Zufuhr abhängen, sondern von Faktoren wie Tagesrhythmus, Geschlecht, Alter, Hormonstatus, inflammatori-schen Prozessen u.v.m.. Es findet also ein ständiger Austausch von zwischen Intra- und Extrazellulär-raum statt (Rükgauer und Kruse-Jarres 2000). **F⁻:** 1,45 mg liegen über den Empfehlungen; neuere Untersuchungen (biosyn) zeigen, dass über fluorhaltige Anästhetika meist eher zuviel Fluor zugeführt wird, so dass für eine kurzfristige parenterale Ernährung eigentlich keine zusätzliche F-Gabe notwendig wäre; allerdings wird angegeben, dass physiologisch wirksam nur die anorganischen Fluor-Verbindungen sind (Heseker 1999) und die organischen so nicht als Fluorzufuhr gewertet werden können. **J⁻:** 0,0015 mg entsprechen ca. 1/90 der Zufuhrempfehlungen; da die lodversorgung der Patienten durch iodhaltige Kontrast- und Desinfektionsmittel meist übernormal ist, dürfte es es dennoch nicht zu einem lodmangel kommen; es stellt sich allerdings die Frage, ob die Zufuhr von Iod auf diesem Wege überhaupt zu empfehlen ist: Bei hoher lodzufuhr besteht die Gefahr eines akuten Anstiegs der Schilddrüsenhormone bei Patienten mit vorausgehender lodmangelernährung. (Eine normal funktionierende Schilddrüse mit ausreichender lodversorgung ist in der Lage, die Absorption und Bildung organischer lodverbindungen zu hemmen (Wolff-Chaikoff-Effekt)). **Cr³⁺:** 0,015 mg entsprechen den Mindestempfehlungen; möglicherweise ist dies aber vor dem Hintergrund neuerer Erkenntnisse (Berger 2004), dass eine Zufuhr von 0,04 mg die Glykämie sowie Insulinämie reduziert, zuwenig, In anderen Untersuchungen (biosyn) wurde allerdings festgestellt, dass es in der Regel nicht zur Cr-Unterversorgung kommt, da viele Lösungen mit Cr kontaminiert sind (Shenkin 2001). **Se⁴⁺:** 0,07 mg liegen innerhalb der Zufuhrempfehlungen; zahlreiche Untersuchungen zeigen aber (z.B. biosyn), dass eine tägliche Gabe von 1 mg von Vorteil sind und nicht zu übernormalen Blutspiegeln führen. **Mo⁴⁺:** 0,025 mg liegen leicht über den Zufuhrempfehlungen; zu Mo treten aber in der Literatur oft widersprüchliche Daten auf, die sich möglicherweise auf Messschwierigkeiten zurückführen lassen; z B. können hohe Werte schon durch Kontamination bei der Blutabnahme mit Stahlkanülen erzeugt werden (Versieck 1983) **Co:** Eine separate Kobaltzufuhr wird nicht empfohlen, da es über Cobalamin = Vitamin B12 zugeführt wird. | | | | | | | | | |

Daher kann geschlussfolgert werden, dass die derzeit bestehende routinemäßig verabreichte Spurenelementsubstitution zu einer Disbalance zwischen Mengen- und Spurenelementen im Blut führt. Durch den Einsatz der Inzolen-Präparate kam es zu extremen Überdosierungen von Mengen- als auch Spurenelementen - wie beispielsweise von Magnesium, Kupfer und Mangan. Im Gegensatz dazu wurde Eisen, Selen und Zink nicht bzw. nicht im ausreichendem Maß zugeführt. Der entscheidendste Nachteil der Inzolen-Präparate ist demzufolge, dass Mengen- und Spurenelemente nicht separat dosiert werden können. Einerseits ist es in der klinischen Routine unmöglich, bei jedem Patienten täglich jedes Spurenelement im Blut zu bestimmen und die Spurenelementsupplementierung darauf auszurichten. Auf der anderen Seite sollte die Zufuhr von Mengen- und Spurenelementen so individuell wie möglich durchgeführt werden. Daher ist es anzustreben, eine insbesondere für den Intensivpatienten; dabei insbesondere für Sepsispatienten, optimal zusammengestellte Spurenelementlösung zu entwickeln, die eine Grundversorgung mit den o.g. essentiellen Elementen gewährleistet und dem erhöhten Bedarf an Selen und/oder Zink gerecht wird.

Der vorliegenden Erfindung lag das technische Problem zugrunde, eine Spurenelemente enthaltende Zusammensetzung für die Ernährung anzugeben, die den tatsächlichen Bedarf des zu Ernährenden an den Spurenelementen Selen bzw. Zink besser deckt als dies mit den Zusammensetzungen aus dem Stand der Technik möglich ist.

Dieses Problem wird erfindungsgemäß gelöst durch die Ausführungsformen 1-13, wie folgt:
1. Spurenelementzusammensetzung für die Ernährung in Form eines Elektrolytkonzentrats, dadurch gekennzeichnet, dass eine Tagesdosis der Zusammensetzung 0.5-2mg Selen und 10 mg-100 mg Zink als Spurenelemente enthält, und wobei Eisen als Spurenelement nicht enthalten ist.
2. Zusammensetzung nach Ausführungsform 1, dadurch gekennzeichnet, dass eine Tagesdosis der Zusammensetzung 1-2 mg Selen und 30 mg-100 mg Zink als Spurenelemente enthält.
3. Zusammensetzung nach Ausführungsform 1 oder 2, dadurch gekennzeichnet, dass die zusammensetzung als Infusionslösung vorliegt.
4. Zusammensetzung nach Ausführungsform 3, dadurch gekennzeichnet, dass die Infusionslösung in einer für die parenterale Verabreichung geeigneten Form vorliegt.
5. Zusammensetzung nach einer der Ausführungsformen 1 - 4, dadurch gekennzeichnet, dass sie als wässrige Lösung für Injektionszwecke vorliegt.
6. Zusammensetzung nach einer der Ausführungsformen 1-5, dadurch, gekennzeichnet, dass weitere Spurenelemente vorliegen, die ausgewählt werden aus Chrom, Fluor, Jod, Kupfer, Mangan und Molybdän.
7. Zusammensetzung nach einer der Ausführungsformen 1-6, dadurch gekennzeichnet, dass die Zusammensetzung als 10 ml Infusionslösung formuliert ist.
8. Verabreichungseinheit einer Zusammensetzung nach einer der Ausführungsformen 1-7, dadurch gekennzeichnet, dass sie als wässrige Lösung in einer Ampulle vorliegt.
9. Verwendung von Selen und Zink zur Herstellung einer Zusammensetzung zur Ernährung von Intensivpatienten, insbesondere von Sepsis-Patienten, dadurch gekennzeichnet, dass die Tagesdosis an Selen in dem Bereich von 0,5 mg - 2 mg und die Tagesdosis an Zink in dem Bereich von 10 mg -100 mg liegt und dass bei der Verabreichung auf das Spurenelement Eisen verzichtet wird.
10. Verwendung nach Ausführungsform 9, dadurch gekennzeichnet, dass die Tagesdosis an Selen mindestens 1 mg und die Tagesdosis an Zink mindestens 30 mg beträgt.
11. Verwendung nach Ausführungsform 10, dadurch gekennzeichnet, dass die Spurenelemente parenteral verabreicht werden.
12. Verwendung nach einer der Ausführungsformen 9-11, dadurch gekennzeichnet, dass weitere Spurenelemente ausgewählt aus Chrom, Fluor, Jod, Kupfer, Mangan und Molybdän verabreicht werden.
13. Verwendung nach einer der Ausführungsformen 10-12, dadurch gekennzeichnet, dass die Spurenelemente über einen Zeitraum von mindestens 3 Tage, vorzugsweise mindestens 5 Tage, verabreicht werden.
14. Spurenelementzusammensetzung zur Verwendung in der Ernährung eines Intensivpatienten in der Form eines Elektrolylkonzentrats, dadurch gekennzeichnet, dass eine Tagesdosis der Zusammensetzung 0,5 - 2 mg Selen und 10 mg - 100 mg Zink als Spurenelemente enthält, wobei Eisen als Spurenelement nicht enthalten ist, und wobei die Zusammensetzung als Infusionslösung vorliegt.

Die erfindungsgemäße Zusammensetzung unterscheidet sich vom Stand der Technik dadurch, dass die erfindungsgemäße Zusammensetzung kein Eisen als Spurenelement enthält

Die erfindungsgemäße Zusammensetzung enthält eine wesentlich höhere Konzentration an Zink bzw. Selen (bezogen auf die übrigen Spurenelemente) als die Präparate aus dem Stand der Technik. Während im Stand der Technik die Konzentration von Selen zu Chrom in dem Bereich von 2:1 liegt, beträgt diese bei den erfindungsgemäßen Zusammensetzungen vorzugsweise mindestens 5:1, besonders bevorzugt mindestens 10:1. Für Zink beträgt das Verhältnis Zn : Cr im Stand der Technik G.300:1, während bei der vorliegenden Erfindung das Verhältnis vorzugsweise mindestens 1000:1, besonders bevorzugt mindestens 3000:1 beträgt.

Eisenmangel wird vor allem durch hohe Blutverluste hervorgerufen, die trauma- bzw. operativ-bedingt, vor allem durch eine Bluttransfusion ausgeglichen werden müssen. Da Bakterien für ihr Wachstum freies Eisen benötigen, stellt der Abfall des Serumeisens während Infektionen und Traumen durch eine zytokinvermittelte Zunahme der Eisenspeicher (Ferritin) eine Komponente der unspezifischen Immunabwehr dar. Aus diesen Gründen sollte auf die Substitution von freiem Eisen in der Akutphase und bei Infektionen verzichtet werden.

Bei einer weiteren bevorzugten Ausführungsform liegt die erfindungsgemäße Zusammensetzung als Infusionslösung vor.

Das in der Zusammensetzung enthaltene Selen bzw. Zink kann in verschiedenen Formen vorliegen. Als Selenhaltige Substanzen können sowohl anorganische wie organische Selenverbindungen eingesetzt werden. Zu den anorganischen Selenverbindungen zählen z.B. Selenit und Selenat, wobei Natriumselenit besonders bevorzugt ist. Zu den organischen Selenverbindungen zählen Selenomethionin, Selenocystein sowie Verbindungen der folgenden allgemeinen Formel: R₁-(Se)ₙ-R₂, worin n eine ganze Zahl von 1-8 bedeutet; R₁ und R₂ können gleich oder verschieden sein und stellen entweder Wasserstoff oder eine Alkylgruppe mit 4-12 Kohlenstoffatomen dar, wobei die Alkylgruppen mit mindestens einer Carboxylgruppe substituiert sein können.

Als Zinkhaltige Verbindungen können organische und anorganische Verbindungen verwendet werden: Zu den organischen Zinkverbindungen zählen z.B. die Zn-Salze von Aminosäuren wie z.B. Zn-Aspartat. Als anorganische Verbindung ist Zinkchlorid bevorzugt. Neben den Spurenelementen enthält die Zusammensetzung vorzugsweise Wasser für Injektionszwecke sowie gegebenenfalls Salzsäure.

Bei einer weiteren bevorzugten Ausführungsform liegt die erfindungsgemäße Zusammensetzung als Infusionslösung vor, die für die parenterale Verabreichung geeignet ist.

Bei einer weiteren bevorzugten Ausführungsform wird die Zusammensetzung als Konzentrat bereitgestellt, das 0,004 mg/ml - 0,2 mg/ml Selen und/oder 1,0 mg/ml - 10 mg/ml Zink enthält. Dieses Konzentrat wird vor dem Verabreichen verdünnt z.B. mit einer kompatiblen Infusionslösung, wobei das Konzentrat ungefähr 1: 10 bis 1 : 50 mit der kompatiblen Infusionslösung verdünnt wird.

Bei einer weiteren bevorzugten Ausführungsform enthält die Zusammensetzung weitere Spurenelemente, die ausgewählt werden aus Chrom, Fluor, Jod, Kupfer, Mangan und Moleden. Vorzugsweise enthält die Zusammensetzung jedes dieser weiteren Spurenelemente, wobei die Konzentrationen dieser Spurenelemente dem Stand der Technik entsprechen wie bspw. in dem gewerblich erhältlichen Präparat Tracutil^{®}.

Bei einer weiteren bevorzugten Ausführungsform wird die erfindungsgemäße Zusammensetzung als Infusionslösung formuliert, die vorzugsweise als 10 ml Verabreichungseinheit formuliert wird. Vorzugsweise wird jede Verabreichungseinheit in Form einer Ampulle für Injektionszwecke bereitgestellt:

Die erfindungsgemäße Zusammensetzung wird vorzugsweise für die Ernährung von Intensivpatienten verwendet. Dabei wird eine Tagesdosis an Selen in dem Bereich von 0,5 mg - 2,0 mg und eine Tagesdosis an Zink in dem Bereich von 10 mg - 100 mg verabreicht. Diese Angaben beziehen sich jeweils auf die Menge an Selen bzw. Zink, so dass bei Einsatz von entsprechenden Selen- bzw. Zinkverbindungen eine entsprechend höhere Menge zu verabreichen ist. So entspricht bspw. 1 mg Selen einer Menge von 3,331 mg Natriumselenit x 5 H₂O. Entsprechend muß zum Verabreichen von 30 mg Zink eine Menge von 62,542 mg Zinkchlorid verabreicht werden.

Intensivpatienten sind z.B. durch folgende Parameter gekennzeichnet: drohender Herkreislauf-Stillstand, Atmungsinsuffizienz und/oder Koma; weitere Beurteilungskriterien befidnen sich z.B. Leuwer M. et al.: Checkliste Interdisziplinäre Intensivmedizin, Thieme Verlag Stuttgart, 1999.

Bei einer besonders bevorzugten Ausführungsform beträgt die Tagesdosis an Selen mindestens 0,5 mg, vorzugsweise 1 mg, und die Tagesdosis an Zink mindestens 10 mg, vorzugsweise, mindestens 30 mg.

Bei einer weiteren bevorzugten Ausführungsform erfolgt das Verabreichen der erfindungsgemäßen Zusammensetzung über mindestens 3 Tage mit den jeweils oben angegebenden Tagesdosen an Zink und Selen, wobei insbesondere eine Verabreichung über mindestens 5 Tage bevorzugt ist.

Vorzugsweise wird die erfindungsgemäße Zusammensetzung vor dem Verabreichen mit einer kompatiblen Infusionslösung verdünnt. So können bspw. 10 ml der Zusammensetzung, die einer Tagesdosis entsprechen, in mindestens 250 ml einer kompatiblen Infusionslösung verabreicht werden. Das Verabreichen dieser Tagesdosis erfolgt vorzugsweise über einen Zeitraum von ungefähr 2 bis 3 Stunden.

Die erfindungsgemäße Zusammensetzung findet vorzugsweise Anwendung bei der Ernährung von Intensivpatienten, insbesondere bei Sepsispatienten. Es hat sich gezeigt, dass mit Hilfe der erfindungsgemäßen Zusammensetzung die Mortalitätsrate bei Sepsispatienten deutlich gesenkt werden kann. Dabei ist es insbesondere von Bedeutung, dass der Selengehalt der Zusammensetzung höher ist als im Stand der Technik.

Die Anforderungen an die Herstellung von Infusionslösungen ist bspw. beschrieben im European Pharmacopoeia, 4^{th} Edition, Supplement 4.6, herausgegeben von EDQM, 2003; S.3933.

### Selen:

### Pharmakodynamik

### Wirkungsmechanismus und Dosis-Wirkungsbeziehung:

Selen ist für die Aktivität einer Reihe von Enzymen von Bedeutung. Bisher konnten rund 20 Selenoproteine identifiziert werden. Zu den wichtigsten selenabhängigen Enzymen, die bisher beim Menschen nachgewiesen wurden, gehören: vier Glutathionperoxidasen (Zytosolische GSH-Px, Plasma GSH-Px, Gastrointestinale GSH-Px, Phospholipid-Hydroperoxid-GSH-Px), die Thioredoxinreduktase (TR), die Deiodasen und ein im Plasma gefundenes Selenbindungsprotein - Selenoprotein P. In allen Selenoproteinen liegt Selen in Form der Aminosäure Selenocystein vor.

Im Jahr 1973 wurde Selen als Bestandteil der Glutathionperoxidase nachgewiesen und damit ein entscheidender Beweis der Essentialität dieses Spurenelements geliefert. Die Glutathionperoxidase konnte inzwischen in allen Geweben von Säugetieren, in denen oxidative Prozesse ablaufen, gefunden werden. Durch die Katalyse von H₂O₂ zu H₂O (a) und von Hydroperoxiden zu entsprechenden Alkoholen (b) wirkt das Enzym protektiv gegen Folgeprodukte reaktiver Sauerstoffverbindungen und ist somit am Lipidperoxidschutz des Organismus beteiligt.

(a) H₂O₂ + 2 GSH → 2 H₂O + GSSG

(b) ROOH + 2 GSH → ROH + H₂O + GSSG

In zellulären oder subzellulären Modellsystemen wurde gezeigt, dass die Integrität zellulärer und subzellulärer Membranen entscheidend von der Intaktheit des Glutathionperoxidase-Systems abhängt. Selen als Bestandteil der Glutathionperoxidase kann somit wie beschrieben die Lipidperoxidationsrate und daraus resultierende Membranschäden senken und im weiteren Genschädigung, Mutationen und schließlich den Zelluntergang verhindern.

Die selenhaltige Glutathionperoxidase beeinflusst außerdem den Leukotrien-, Thromboxan- und Prostazyklinstoffwechsel und hat damit Bedeutung bei Entzündungsvorgängen.

In Form von Natriumselenit hat Selen neben der Funktion als Selenlieferant noch eine weitere: Natriumselenit geht unter hypoxisch-azidotischen Bedingungen in selenige Säure über und ist so in der Lage, organische Oxyl- und Hydroxylradikale zu ROOH bzw. HOOH zu oxidieren. Es können daher enzymunabhängig spontan freie Radikale gebunden werden. Dies erklärt die Wirksamkeit von Natriumselenit vor allem in der Frühphase der Sepsis. Es ist nicht davon auszugehen, dass die Synthese der Selenoproteine bei Patienten im Postaggressionstoffwechsel optimal abläuft. So ist ein Anstieg der Glutathionperoxidase auch erst ab dem dritten Tag der Selentherapie zu beobachten, trotzdem setzt die Wirksamkeit von Natriumselenit bereits am ersten Tag ein.

Bis zu 70 % des Plasmaselens sind an Selenoprotein P gebunden - ein Protein dessen Funktion noch nicht eindeutig geklärt ist. Es existieren jedoch eindeutige Untersuchungen, die Selenoprotein P zum einen als extrazelluläres Antioxidans mit einer Phospholipid-Hydroperoxid-Glutathionperoxidase-Funktion identifizieren. Zum anderen übt Selenoprotein P offenbar eine Transportfunktion aus. So ist bekannt, dass Selenoprotein P 35 Quecksilber-Selen-Komplexe (mit je 100 Quecksilber-Selen-Molekülen) binden und über den Urin ausscheiden kann.

Die Umwandlung des Schilddrüsenhormons Tetraiodthyronin (T4) zu dem biologisch aktiven Triiodthyronin (T₃) ist die Aufgabe der Enzyme Typ-I- und Typ-II-Deiodasen. In einer Reihe von Untersuchungen konnte nachgewiesen werden, dass es sich bei der in der Leber, Niere und Schilddrüse vorkommenden Typ-I-Deiodase um ein Selenoprotein handelt, das in seinem aktiven Zentrum ein Atom Selen pro Molekül enthält. Dieses Enzym katalysiert sowohl die 5'- als auch die 5-Monodeiodation und bildet somit sowohl T₃ als auch rT₃. Es erzeugt 80 % des im Plasma befindlichen T₃. Die Typ-II-Deiodase, ebenfalls ein Selenoprotein befindet sich vor allem im Gehirn, im braunen Fettgewebe und in der Plazenta und bildet dort Thyroxin T₃. Die dritte Deiodase (Typ III) katalysiert die 5-Monodeiodation, d.h. durch dieses Enzym wird nur rT₃ gebildet. Mit diesem Mechanismus kann durch eine erhöhte Bildung von rT₃ der Stoffwechsel bei z.B. Hunger oder schwerer Erkrankung gedrosselt werden (Low-T₃-Syndrom). Auch dieses Enzym wurde als Selen-haltiges Protein beschrieben.

Selen spielt weiterhin bei der Immunabwehr eine bedeutende Rolle. Tierversuche deuten darauf hin, dass entsprechende Selen-Dosen das Immunsystem positiv beeinflussen. Durch Selen-Gaben konnten eine vermehrte Antikörperbildung, gesteigerte Proliferationsraten der T- und B-Lymphozyten sowie eine gesteigerte Zytotoxizität der T-Lymphozyten und Natural Killer-Zellen erreicht werden. Selen-Gaben von täglich 200 µg über acht Wochen erhöhten beim Menschen die Ausbildung des Interleukin-2-Rezepors, die Lymphozytenproliferation sowie die zellzerstörende Wirkung der T-Lymphozyten und Natural Killer-Zellen (Kiremidjian-Schumacher et al. 2000, 1994, Roy et al. 1994). Beim Menschen führte eine Supplementierung von täglich 200 µg Selen als Natriumselenit innerhalb von drei Wochen zu einem signifikanten Anstieg der Natural Killer-Zellen. Ferner wurde durch Anreicherung des Tafelsalzes mit 15 ppm Natriumselenit in einer Gruppe von 20.847 Probanden über fünf Jahre die Inzidenz von primärem Leberzellkrebs (versus Kontrolle von 109.624) um 40% reduziert.

Selen ist auch an der Detoxifikation von Schwermetallen wie Cadmium, Quecksilber, Blei und Arsen beteiligt. Ein Mechanismus besteht darin, dass dabei biologisch inaktive Selenide gebildet werden und die Bindung von Quecksilber und Cadmium an lebenswichtige Proteine verhindert wird.

### Toxikologie und Nebenwirkungen:

An einigen Versuchstieren wurden für Natriumselenit die LD₅₀-Werte bestimmt. Nach intravenöser Verabreichung betrug die LD₅₀ für Mäuse 2,2 mg Selen/kg KG, für Ratten 5,7 mg/kg KG und für Kaninchen 0,9 mg/kg KG. Nach peroraler Verabreichung wurden für Kaninchen 2,25 mg/kg KG, für Meerschweinchen 2,3 mg/kg KG und für Mäuse 3,2 - 3,5 mg/kg KG als LD₅₀ ermittelt.

Teratogene und embryotoxische Wirkungen des Selens konnten im Tierexperiment nachgewiesen werden.

Die meisten Studien zur Wirkung von Selenverbindungen auf tierexperimentell erzeugte Tumoren zeigen eine hemmende Wirkung von Selenverbindungen auf das Tumorwachstum.

Die akute Toxizität für Natriumselenit (verschiedene Versuchstiere) liegt bei 1-5 mg Selen pro kg KG, die chronische Toxizität bei 3-7 mg Selen/Tag. Erste Vergiftungssymptome treten bei 1,05 mg Selen/l Vollblut auf. Akute Symptome sind vermehrtes Schwitzen, Erbrechen, Muskelspasmen, Herzrhythmusstörungen; chronische Symptome sind Haarausfall, brüchige Fingernägel, Hautveränderungen, Störungen des Nervensystems. Die Symptome sind in den meisten Fällen reversibel und verschwinden innerhalb von ein bis zwei Wochen.

Beim Menschen sind akute Selenintoxikationen selten beschrieben und lassen sich in den meisten Fällen auf die Einwirkung von Selenstäuben oder Selenwasserstoff zurückzuführen.

Eine akute Selenvergiftung wurde z.B. bei zwei Kindern beobachtet, die von einem Waffenreinigungsmittel getrunken hatten, das ca. 1,8 % selenige Säure enthielt. Die Kinder hatten auf diese Weise 10 - 15 ml des Mittels, entsprechend ca. 110 mg = 110.000 µg Selen, aufgenommen und verstarben an den Folgen.

Chronische Selenvergiftungen des Menschen werden vor allem in Gebieten mit extrem hohen Selengehalten der Böden beobachtet. Die Selen-Aufnahme in diesen Gegenden wird auf täglich 3 - 7 mg Selen geschätzt.

In einem Bericht über eine chronische Vergiftung mit Natriumselenit wird der Fall eines 62-jährigen Mannes beschrieben, der über mehr als zwei Jahre täglich 2 mg Natriumselenit, entsprechend 900 µg Selen, eingenommen hatte. Es wurde ein knoblauchartiger Atemgeruch festgestellt. Seine Fingernägel waren verdickt und brüchig, wuchsen jedoch nach Absetzen der Selen-Einnahme normal nach. Es wird weiterhin der Fall einer 57-jährigen Frau geschildert, die ca. zwei Monate lang täglich 27 mg Selen in Form von Natriumselenit eingenommen hatte und an Mattigkeit, Übelkeit, Erbrechen, Depressionen, üblem Mundgeruch, Haarausfall und abfallenden Nägeln litt. Die Vergiftungserscheinungen waren möglicherweise durch die gleichzeitige Einnahme von Vitamin C gemildert worden. Vier Personen nahmen über 20 - 40 Tage täglich 2 mg Selen in Form von Natriumselenit auf, was keine Zeichen einer Selenvergiftung zur Folge hatte. Nach über einjähriger peroraler Gabe von täglich 50 µg Selen pro kg Körpergewicht als Natriumselenit wurden bei Patienten mit neuronaler ceroider Lipofuscinose ebenfalls keine Symptome einer Selenvergiftung beobachtet.

Selenvergiftungen gehen mit erhöhten Selenkonzentrationen im Blut und im Urin einher. Beim Menschen können Symptome ab einem Selengehalt von 1000 µg/l Vollblut auftreten.

Im Hinblick auf eine Langzeit-Anwendung (länger als drei Monate) wird eine Dosierung von täglich bis zu 500 µg Selen, in Form anorganischer Salze wie Natriumselenit bis zu 550 µg Selen als unbedenklich angesehen. Spätere Empfehlungen lauten auf höchstens 50 µg Selen/kg KG (3500 µg/70 kg KG) als orale Einmalgabe bzw. höchstens 5 µg Selen/kg KG (350 µg/70kg KG) bei längerfristiger Anwendung. Die momentan gültige Empfehlung der WHO (1996) für die sichere maximale Aufnahme über einen längeren Zeitraum liegt bei 400 µg Selen täglich. Diese stellt einen Kompromiss dar, der die Unterschiede mit einbezieht, aber nur für den gesunden Menschen ohne Selenmangelsymptome und ohne größere Belastungssituationen Gültigkeit hat. Menschen mit sehr niedrigen Selenspiegeln, konsumierenden Erkrankungen, chronischen Entzündungen oder Infektionen bzw. Erkrankungen, die mit einer überschießenden Radikalproduktion einhergehen, sind hier nicht berücksichtigt und müssen gesondert betrachtet werden. Die notwendigen - und deshalb auch verträglichen - Dosierungen liegen hier sehr viel höher.

### Pharmakokinetik

### Verteilung und Metabolismus:

Die Absorption von Selen hängt von seiner chemischen Bindungsform und der Zusammensetzung der Nahrung ab. In Fleisch, Fisch, Eiern, Pflanzen und Hefe liegt Selen gebunden an Aminosäuren vor (Selenomethionin und Selenocystein) und wird mittels des aktiven Aminosäuren-Transportmechanismus vorwiegend im Dünndarm absorbiert. Selenomethionin muss, bevor es in Selenoproteine eingebaut werden kann, in mehreren Schritten umgebaut werden. Im Intermediärstoffwechsel konkurriert es mit Methionin und wird daher auch unspezifisch in andere Proteine, insbesondere Albumin integriert (Waschulewski und Sunde 1988), wodurch es zum großen Teil für die Selenoproteine nicht mehr zur Verfügung steht. Natriumselenit dagegen passiert die Darmschleimhaut durch passive Diffusion. Die Resorptionsrate beträgt zwischen 44 % und 80 %. Nach oraler Gabe von 1 mg Natriumselenit wurde übereinstimmend in mehreren Untersuchungen ein mittlerer Anteil 62 ± 14 % absorbiert.

Im Blut wird Selenit hauptsächlich von den Erythrozyten aufgenommen. Selenwasserstoff dient als zentraler Selenpool für die Ausscheidung und für den gezielten Einbau in Selenoproteine. In dieser reduzierten Form wird Selen an Plasmaproteine gebunden und in die Leber und andere Organe transportiert. Der von der Leber ausgehende plasmatische Sekundärtransport in die Glutathionperoxidase-synthetisierenden Zielgewebe geschieht wahrscheinlich in Form eines Selenocystein-haltigen P-Selenoproteins. Schließlich wird eine Selenocysteinyl-tRNA gebildet, indem zunächst eine Serinyl-tRNA phosphoryliert und dann das Selen im Austausch gegen die Phosphatgruppe an Stelle des Schwefels eingebaut wird. Der Einbau von Selenocystein in Selenoproteine erfolgt hochspezifisch an eine definierte Position der Aminosäuresequenz mit Hilfe der Selenocysteinyl-tRNA am UGA-Codon der m-RNA. Der Verlauf der Selenoprotein-Biosynthese ist sowohl für Prokaryonten als auch für Eukaryonten nahezu aufgeklärt. Überschüssiger Selenwasserstoff wird über Methylselenol und Dimethylselenid zum Trimethyl-Selenium-Ion, dem hauptsächlichen Ausscheidungsprodukt, metabolisiert.

Der Selengehalt im Blut variiert stark und ist direkt korreliert mit der Selenaufnahme aus der Nahrung, wobei die höchsten Werte in den Thrombozyten gemessen werden. Aber auch in den Erythrozyten sind die Mengen an Selen und dem Selen-haltigen Enzym Glutathionperoxidase gegenüber dem Serum erhöht. Durch die stark variierenden Selenkonzentrationen im Blut ist es problematisch, Normalwerte festzulegen. Einige Autoren geben Plasmaspiegel von 40-190 µg/l an. Nach neueren Ergebnissen liegen beim Menschen häufig gemessene Selenkonzentrationen bei 90-130 µg/l im Vollblut bzw. bei 75-120 µg/l im Serum. Bis zu einer Selenkonzentration von ca. 160 µg/l im Vollblut besteht eine enge Korrelation zwischen den Selenwerten und der Aktivität der Glutathionperoxidase in den Erythrozyten. Im Interesse einer maximalen Aktivität der Glutathionperoxidase und damit eines ausreichenden Schutzes vor oxidativen Schädigungen ist ein genügend hoher Selengehalt im Blut anzustreben. Ist eine ausreichende Selenaufnahme über die Nahrung nicht gewährleistet, kann mit z.B. Natriumselenit supplemiert werden. Dies gilt insbesondere bei klinisch nachgewiesenem Selenmangel.

Die Gesamtmenge an Selen im menschlichen Körper liegt zwischen 4 mg und 20 mg. Im menschlichen Körper enthalten die Schilddrüse, die Leber und die Nieren die höchsten Konzentrationen an Selen.

### Ausscheidung:

Die Ausscheidung von Selen erfolgt beim Menschen je nach applizierter Dosis über die Fäzes, den Urin oder über die Lunge. Der Weg der Exkretion hängt von der Selenaufnahme und vom Selenstatus ab. Das mit der Nahrung aufgenommene Selen verlässt zu 50 - 70 % den Organismus renal. Die in Europa analysierten Harnkonzentrationen liegen unter 30 - 40 µg Selen/l. Bei steigenden Selenaufnahmen verschiebt sich die Selenausscheidung deutlicher von den Faeces zum Urin, ein Tatbestand, der die Rolle der Niere bei der homöostatischen Regulation des Selen-Haushaltes unterstreicht. Nach Aufnahme der den Bedarf übersteigenden Selenmenge wird das Trimethylselenonium zum dominierenden Urinmetabolit.

Ein weiterer Detoxifikationsmetabolit des Selens ist das in der Atemluft ausgeschiedene Dimethylselenid. Die Ausscheidung über die Atemluft und die Haut ist jedoch unter physiologischen Bedingungen zu vernachlässigen. Erst bei Verabreichung sehr hoher bzw. toxischer Dosen wird zusätzlich knoblauchartig riechendes Dimethylselenid abgeatmet.

Die Selenausscheidung nach intravenöser oder oraler Gabe läuft in drei Phasen ab. Bei oraler Gabe von 10 µg in Form von [⁷⁵Se] Natriumselenit wurden in den ersten zwei Wochen 14 - 20 % der absorbierten Dosis an Selen über den Urin ausgeschieden, während praktisch keine Ausscheidung über die Lunge oder die Haut festgestellt werden konnte. Die Gesamtkörperretention von Selen nahm triphasisch ab mit einer Halbwertszeit von 0,7 - 1,2 Tagen in der 1. Phase, 7-11 Tagen in der 2. Phase und 96-144 Tagen in der 3. Phase. Die Selenkonzentration verminderte sich in Leber, Herz und Plasma schneller ab als im Skelettmuskel oder in den Knochen.

Von einer intravenös verabreichten Dosis von [⁷⁵Se] Natriumselenit wurden innerhalb der ersten 24 Stunden 12 % ausgeschieden. Weitere 40 % wurden mit einer biologischen Halbwertszeit von 20 Tagen eliminiert. Die Halbwertszeit der dritten Phase wurde mit 115 Tagen bestimmt.

Bei einem direkten Vergleich zwischen oraler und intravenöser Verabreichung einer physiologischen Dosis an [⁷⁴Se] Natriumselenit wurden nach intravenöser Gabe von 82 µg Selen in Form von Natriumselenit in den ersten 24 Stunden 18 % der Dosis, nach peroraler Gabe 12 % der absorbierten Dosis zusammen mit metabolisch ausgetauschtem Körper-Selen über den Harn ausgeschieden. Danach verläuft die Ausscheidung für beide Applikationsarten gleichartig. Oral und parenteral appliziertes Natriumselenit ist bei gesunden Probanden vergleichbar.

### Zink

### Pharmakodynamik

### Wirkungsmechanismus und Dosis-Wirkungsbeziehung:

Zink, das mengenmäßig bedeutendste Spurenelement, ist essentiell für die als katalytische und regulatorische Funktion von mehr als 200 Enzymen. Zink-Metalloenzyme finden sich in 6 Enzymklassen (Oxidoreduktasen, Transferasen, Hydrolasen, Lyasen, Isomerasen, Ligasen) und greifen damit in alle Stoffwechselvorgänge (Proteine, Kohlenhydrate, Fette, Nukleinsäuren, etc.) ein.

Beispielsweise im Stoffwechsel von Proteinen und Nukleinsäuren erfüllt Zink zahlreiche Aufgaben wie z.B. die Stabilisierung der Struktur von DNA, RNA, Ribosomen oder als Bestandteil von Schlüsselenzymen der Nukleinsäuresynthese (z.B. DNA-Polymerasen). Daneben gibt es zahlreiche physiologisch relevante Interaktionen zwischen Zink und verschiedenen Hormonen (z.B. Testosteron, adrenale Kortikoide, Insulin, Wachstumshormon), wobei sowohl Produktion, Speicherung und Sekretion als auch Hormon-Rezeptor-Interaktionen involviert sein können.

Zink spielt auch eine Rolle bei der Aufrechterhaltung von Struktur und Funktion der Biomembranen. Es ist unentbehrlich für den Fettstoffwechsel, die Funktion der Sinnesorgane (Geschmackswahrnehmung), die Aufrechterhaltung der Immunfunktionen. In pharmakologischen Konzentrationen wirkt Zink an isolierten Zellsystemen antioxidativ (Superoxiddismutase).

Zink nimmt eine wesentliche Rolle in der Wundheilung, für das Wachstum und im Rahmen der Fortpflanzung ein. Grund hierfür ist seine o.g. Wirkung im Nukleinstoffwechsel. Ferner ist ein ausreichender Zinkspiegel wichtig für die Speicherung von Insulin und die Förderung einer positiven N-Bilanz.

Zu starken Zinkverlusten kommt es besonders bei Operationen im Darmbereich und bei gastroenterologischen Erkrankungen mit Darmfisteln. Zinkmangel ist u.a. bei parenteraler Ernährung, Behandlung mit Chelatbildnern und großflächigen Verbrennungen beobachtet worden.

Entsprechend den Aufgaben, die Zink im Stoffwechsel hat, sind die Zinkmangelsyndrome vielfältig:
- Allgemeinsymptome: Wachstumsstillstand (Kinder durch Knochenwachstumsdepressionen und einem verminderten Einbau von Aminosäuren in die Muskulatur); Gewichtsverlust (Erwachsene und Kinder) bis zur Kachexie trotz ausreichender Zufuhr von Aminosäuren und Energie, Wundheilungsstörungen, Appetitlosigkeit mit Störung der Geschmacksempfindung, Entwicklungsrückstand der Gonaden, verringerte Glukosetoleranz sowie eine Erhöhung der freien Fettsäuren im Blut
- Gastrointestinaltrakt: Therapieresistente Diarrhöen (oft als Erstsymptom), postoperativ anhaltende Darmatonie
- Haut- und Schleimhäute: Dermatitis, die die Umgebung aller Körperöffnungen befällt, Alopecia areata, Wachstumsstillstand der Nägel (Beau-Reill-Querfurchen), Parakeratosen und entzündliche Veränderungen sämtlicher Schleimhäute
- Zentralnervensystem: Verwirrtheit, Apathie, depressive Verstimmung, Unruhe, Gereiztheit, Agitiertheit und Photophobie
- Fortpflanzung: Frühgeburten, Missbildungen (mutagen, teratogen)
- Plasma: Hypozinkämie (2 - 4 µmol/l, Konzentrationsabfälle, wie sie in Stresssituationen nicht beobachtet werden), Hypoproteinämie, Aktivitätsverminderung der alkalischen Phosphatase, Erniedrigung kurzlebiger Plasmaproteine wie Albumin, Transferrin und Präalbumin :
- Immunsystem: Depression der zellulären Immunität (T-Lymphozyten) erleichtert die Keiminvasion über Haut und Schleimhäute und führt häufig zu Septikämien
- Urin: Trotz Hypozinkämie vermehrte Zinkausscheidung im Urin, besonders bei septischen und katabolen Zuständen
- Acrodermatitis enteropathica: autosomal-rezessiv, gestörte Resorption

Die Empfehlung für die enterale Zinkzufuhr wird für Männern mit 10 mg/d und für Frauen mit 7 mg/d angegeben. Erhöhter Bedarf besteht während des Wachstums und in der Reproduktionsphase sowie in der Schwangerschaft und Stillzeit. Bei größeren gastrointestinalen Flüssigkeitsverlusten, längerer Darmerkrankung oder einer Behandlung mit D-Penicillamin sowie nach anhaltenden katabolen Zuständen sollten 3 µmol/kg (10 - 15 mg) zugeführt werden.

Bei parenteraler Ernährung beträgt die Zink-Zufuhr ca. 5 mg, bei größeren Darmverlusten nimmt man eine Bedarfssteigerung um ca. 2 mg/d an. Die intravenöse Zufuhr kann auch mit 1 µmol/kg KG/d errechnet werden. Dem erhöhten Bedarf bei größeren gastrointestinalen Flüssigkeitsverlusten oder bei Behandlung mit Komplexbildnern sollte durch eine Zufuhr von 3 µmol/kg KG/d Rechnung getragen werden. Die Deutsche Arbeitsgemeinschaft für künstliche Ernährung (DAKE) bzw. die Deutsche Gesellschaft für Ernährungsmedizin (DEGM) gibt für die parenterale Ernährung 1,4 - 4,9 mg/d als Empfehlung.

### Toxikologie und Nebenwirkungen:

Zink ist wenig toxisch, der toxische Bereich beginnt erst bei Gramm-Mengen.

Akut toxische Wirkungen von Zink führen im wesentlichen zu gastrointestinalen Symptomen. Sie entstehen meist durch den Genuss von säurehaltigen Speisen/Getränken, die in verzinkten Behältnissen aufbewahrt wurden.

Per os aufgenommenes Zink hat lokale und systemische Giftwirkungen. Bei Einwirkung hoher Konzentrationen stehen die lokalen Nebenwirkungen im Vordergrund. Die gastrointestinalen Erscheinungen beruhen auf einer lokalen Ätzwirkung (Chlorid > Sulfat > Acetat).

1 bis 2 g ZnSO₄ (225 bis 450 mg Zink) per os führt zu Erbrechen, das einen gewissen Schutz vor resorptiver Vergiftung bietet. 5 g ZnSO₄ bzw. 3 bis 5 g ZnCl₂ (starke Ätzmittel) gelten für den Erwachsenen als tödlich.

Versehentliche intravenöse Infusion von insgesamt 7,4 g Zink (als Sulfat) innerhalb von 60 Stunden endete mit dem Tod eines 72-jährigen Mannes nach 47 Tagen unter unspezifischen Symptomen bei autoptisch nachgewiesener Nierentubulusnekrose und toxischer Leberschädigung.

Fatale Wirkung hatte die versehentliche intravenöse Infusion von 1,5 g Zink über 60 Stunden bei einer Frau. Sie entwickelte Bluthochdruck, Lungenödem, Erbrechen, Gelbsucht und Oligourie mit Nierenschaden, der sich als Nekropsie äußerte.

Ein 16-jähriger Junge, der 2 Tage lang 12 g elementares Zink/d einnahm, entwickelte Leichtsinnigkeit, Lethargie und Schreibschwierigkeiten, begleitet von einem erhöhten Blutzinkspiegel, erhöhten Serumamylase- und -lipaseaktivitäten, wahrscheinlich als Folge der Zinkwirkung auf die Pankreasfunktion.

Die Toxizität von Zink bei wiederholter Verabreichung ist im wesentlichen gekennzeichnet durch einen Kupfermangel und führt zu einer hypochromen Anämie.

In einem anderen Fall führten 150 mg Zink pro Tag über 2 Jahre zu Kupfermangel, der sich durch eine mikrozytäre Anämie, Neutropenie und erniedrigte Plasmaspiegel von Kupfer und Coeruloplasmin äußerte.

Bei einem 13 Monate alten Mädchen, das vom sechsten Lebensmonat an prophylaktisch täglich 16 mg Zink (als Glukonat) und ab einem Jahr täglich 24 mg Zink erhalten hatte, bestand eine chronische Zinkvergiftung mit ausgeprägten Symptomen des Kupfermangels.

20 gesunde junge Männer, die 160 mg Zink/d über 5 Wochen erhielten, hatten normale Werte an Gesamtcholesterin, Triglyceriden und LDL-Cholesterin. HDL-Cholesterin wurde jedoch signifikant reduziert, normalisierte sich aber 7 Wochen nach der Behandlung wieder. Bei gesunden jungen Frauen hatte eine Zinksupplementation mit 100 mg täglich über 8 Wochen jedoch keinen Einfluss auf die Blutfettwerte.

Nach einwöchiger Einnahme von 2-mal täglich 220 mg Zinksulfat zur Akne Behandlung kam es bei einem 15 jährigen Mädchen zu einer Gastritis mit hämorrhagischen Erosionen, die nach Absetzen des Zinkpräparates spontan abheilten.

Insgesamt gesehen werden per os relativ hohe Zinkdosen auch über längere Zeit gut vertragen. Wegen der metabolischen Wechselwirkungen von Zink mit Kupfer, Eisen, Kalzium und Kadmium lässt sich eine unbedenkliche Höchstdosis an Zink aber nicht angeben. Und auch eine mäßig erhöhte Zufuhr, wie sie bei der Substitutionsbehandlung üblich und bei der Selbstmedikation möglich ist, kann einen (latenten) Kupfermangel hervorrufen, die Immunfunktion stören und - vielleicht - das Lipoproteinprofil ungünstig beeinflussen.

### Pharmakokinetik

### Verteilung und Metabolismus:

Die Zink-Resorption erfolgt fast ausschließlich vom Dünndarm aus, sowohl durch passive Diffusion und als auch durch aktiven Transport (cysteinreiches intestinales Protein).

Die homöostatische Regulation der Zinkresorption funktioniert nur bei intakter Schleimhaut. Die Resorption wird wahrscheinlich durch ein Wechselspiel zwischen zwei zinkbindenden Proteinen der Dünndarmmukosa, 1. dem "cysteinreichen intestinalen Protein" (CRIP), dem Zink-Carrier für den Transport über die Mukosazelle, und 2. Metallothionein, dessen Synthese (im Gegensatz zur CRIP-Synthese) durch Zinküberschuss induziert wird, gesteuert. Dadurch kann - in Grenzen - überschüssiges Zink von der Resorption ausgeschlossen werden.

Aus zinkarmer Nahrung wird relativ mehr resorbiert als aus zinkreicher. Für Zink scheint kein spezifischer Speicher zu existieren. Deshalb führt eine drastische Reduktion der alimentären Zufuhr rasch zu einem Mangel.

Die Resorptionsquote hängt vom Bedarf und von der Verfügbarkeit ab. Im Tierexperiment und beim gesunden Menschen lag sie zwischen weniger als 10 und mehr als 90 %). Unter physiologischen Bedingungen werden aus der Nahrung etwa 20 - 30 % resorbiert.

Die Zinkresorption wird vor allem durch Phytat (Inositolhexaphosphat) gehemmt, das reichlich im Getreide enthalten ist (Zn-Phytat- oder sehr schwer lösliche Zn-Ca-Phytat-Komplexe). Weiterhin beeinflusst ein niedriger Proteingehalt sowie in geringerem Umfang auch ein hoher Fasergehalt der Nahrung die Zinkresorption negativ. Eisen, Kupfer und Kalzium hemmen ebenfalls die Resorption von Zink und umgekehrt: Dies gilt für pharmakologische Dosen (Mineralstoffpräparate), aber wahrscheinlich nicht für die in Nahrungsmitteln natürlicherweise enthaltenen Mengen.

Die Zinkresorption wird durch Chelatbildner wie EDTA und bestimmte Aminosäuren, die mit Zink Komplexe bilden (Histidin, Tryptophan, Prolin, Lysin, Glycin, Cystein), Ascorbinsäure und Prostaglandin E2 gefördert. Die Resorption wird durch Picolinsäure, die aus Tryptophan entsteht und mit dem Pankreassaft sezerniert wird sowie durch Citrat, Cystein und Glutamat erhöht.

Das resorbierte Zink wird mit dem Pfortaderblut zur Leber transportiert. Bei intravenöser Injektion verschwindet es mit einer Halbwertszeit von fünf Minuten aus dem Blut in die Leber. Die Aufnahme von Zink in die Zelle erfolgt in Konkurrenz mit Eisen und Kalzium.

Die physiologische Serumkonzentration liegt bei 15,2 ±1,5 µmol/l, wobei diese mit zunehmenden Alter abnimmt. Bei Patienten über 60 Jahren sind 11 µmol/l normal.

Entsprechend seiner Funktion verteilt sich Zink weiter in alle Gewebe. Nach parenteraler Gabe erreicht Zink vorübergehend auch im Gehirn erhebliche Konzentrationen. Die Konzentration im Liquor cerebrospinalis beträgt beim Menschen ein Zehntel der Plasmakonzentration. Bei Zinkmangel fällt die Zinkkonzentration im Gehirn nicht, sie kann sogar steigen. Innerhalb des Zentralnervensystems ist Zink spezifisch angereichert, besonders in den Nervenendigungen von Hippocampus, Kortex und Corpus pineale.

Der Körperbestand an Zink beträgt 20 - 30 mmol (1,5 - 2,5 g).

### Ausscheidung:

Die Exkretion von Zink erfolgt zu 90 % über die Faeces, der Rest wird renal ausgeschieden. Resorbiertes oder parenteral verabreichtes Zink wird beim Menschen mit einer Halbwertszeit von 250 bis 500 Tagen zu drei Vierteln in den Darm (und zwar überwiegend in den Dünndarm) ausgeschieden, vor allem mit dem Pankreassaft und mit der Galle, daneben mit Speichel, Magensaft und über das Darmepithel. Auch in der Galle liegt es zum Teil als Metallothionein vor. Dieses ausgeschiedene Zink kann reabsorbiert werden und in einen enteropankreatischen bzw. einen enterohepatischen Kreislauf eingehen.

Die physiologischen Zinkverl uste über Faeces, Urin und Haut betragen bei Männern 1,4 mg/d, bei Frauen 1 mg/d. Bei Lebererkrankungen oder schweren Verletzungen, Verbrennungen werden über den Urin 1 bis 4 mg/d ausgeschieden. Zu pathologischen Zinkverlusten kommt es bei chronischen Blutungen (z. B. Schistosomiasis, Hakenwurminfektionen), Fisteln (z. B. Morbus Crohn) oder Durchfall. Eine gesteigerte Zinkausscheidung im Urin findet sich auch bei ausgedehnten Verbrennungen, Verletzungen, Hyperalimentation, Leberzirrhose, akuter Pankreatitis, Infektionen, chronischen Nierenerkrankungen, Sichelzellanämie, Aminoazidurie bei totaler parenteraler Ernährung, Hypertoniebehandlung mit Thiazid-Diuretika, Krebsbehandlung mit Cisplatin.

### Die folgenden Beispiele erläutern die Erfindung.

### Darreichungsform aus dem Stand der Technik und der vorliegenden Erfindung im Vergleich

| | |
|---|---|
| Medikation: | erfindungsgemäßes Präparat oder Tracutil^{®} (Vergleichspräparat) |
| Darreichungsform: | Infusionslösung |
| Zusammensetzung: | 1 Ampulle mit 10 ml Infusionslösung enthält folgende arzneilich wirksame Bestandteile |

Vergleich der erfindungsgemäßen Zusammensetzung mit der aus dem Stand der Technik bekannten Zusammensetzung Tracutil^{®}:

**Tabelle Zusammensetzung der Medikation**

| Bestandteil | Menge in mg | | Spurenelement | Menge in mg | |
|---|---|---|---|---|---|
| | Tracutil^{®} | syntrace^{®} plus | | Tracutil^{®} | syntrace^{®} plus |
| Chrom-(III)-chlorid 6H₂O | 0,053 | | Chrom | 0,01 | |
| Natriumfluorid | 1,260 | | Fluor | 0,57 | |
| Kaliumiodid | 0,166 | | Iod | 0,13 | |
| Kupfer-(II)-chlorid 2H₂O | 2,046 | | Kupfer | 0,76 | |
| Mangan-(II)-chlorid 4H₂O | 1,979 | | Mangan | 0,55 | |
| Natriummolybdat 2H₂O | 0,0242 | | Molybdän | 0,01 | |
| Eisen-(II)-chlorid 4H₂O | 6,958 | - | Eisen | 1,95 | - |
| Natriumselenit 5H2O | 0,0789 | 3,331 | Selen | 0,02 | 1,00 |
| Zinkchlorid | 6,815 | 62,542 | Zink | 3,27 | 30,00 |
| | | | | | |
| sonstige Bestandteile: | Salzsäure, Wasser für Injektionszwecke | | | | |

Neben der erhöhten Konzentration an Selen und Zink, wurde als weitere Änderung im Hinblick auf das intensivmedizinische Patientengut das zweiwertige Eisen bei vorliegender Erfindung plus mit folgender Begründung weggelassen. Eisenmangel wird vor allem durch hohe Blutverluste hervorgerufen, die trauma- bzw. operativ-bedingt, vor allem durch eine Bluttransfusion ausgeglichen werden müssen. Da Bakterien für ihr Wachstum freies Eisen benötigen, stellt der Abfall des Serumeisens während Infektionen und Traumen durch eine zytokinvermittelte Zunahme der Eisenspeicher (Ferritin) eine Komponente der unspezifischen Immunabwehr dar. Aus diesen Gründen sollte auf die Substitution von freiem Eisen in der Akutphase und bei Infektionen verzichtet werden. Zudem kann der Fachinformation von Tracutil^{®} dem Punkt Nebenwirkungen entnommen werden, dass in Einzelfällen von anaphylaktischen Reaktionen auf parenteral verabreichtes Eisen berichtet wurde.

Das folgende Schema zeigt den Aufbau dieses Vergleichsversuchs.

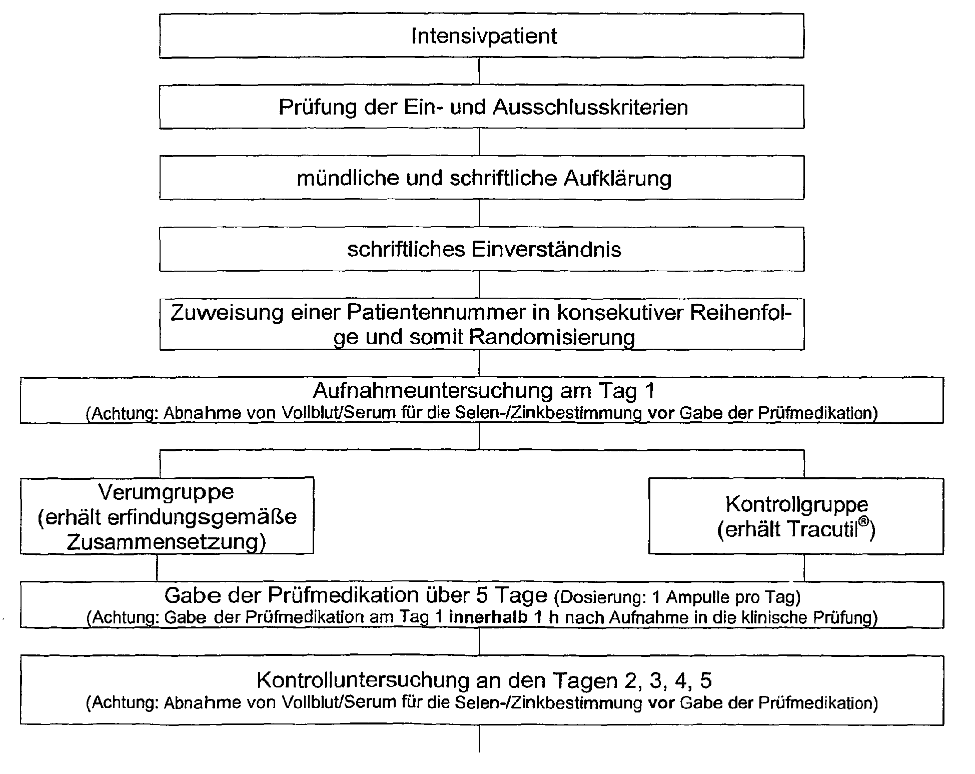

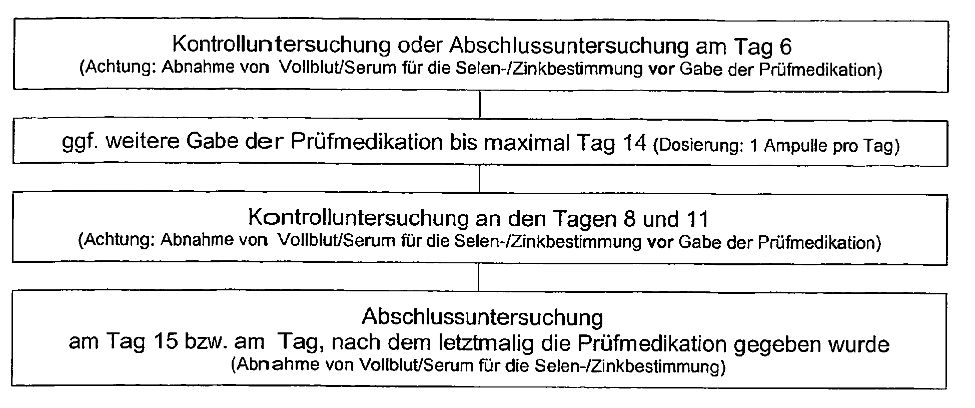

### Dosierung und Art der Anwendung

Über 5 Tage wird pro Tag eine 10 ml-Ampulle der Medikation verabreicht. Die Infusion der ersten Ampulle muss innerhalb der ersten Stunde nach Einschluss des Patienten in die klinische Prüfung beginnen. An den folgenden Tagen müssen weitere Infusionen stets vor 12 Uhr und pro Patient immer zur selben Zeit (± 1 Stunde) erfolgen.

### Beispiel:

Sonntag - 21.00 Uhr Aufnahme auf der Intensivstation
Montag - 17.30 Uhr erste Gabe der Medikation (Tag 1)
Dienstag - 8.00 Uhr Gabe der Medikation (Tag 2)
Mittwoch - 8.30 Uhr Gabe der Medikation (Tag 3)
Donnerstag - 7.30 Uhr Gabe der Medikation (Tag 4)
Freitag - 8.00 Uhr Gabe der Medikation (Tag 5)

Sollten die Intensivpatienten länger als 5 Tage parenteral ernährt werden, so wird die Medikation bis maximal Tag 14 nach o.g. Schema verabreicht.

Die Medikation wurde verdünnt appliziert. Es wurden 10 ml der Prüfmedikation (1 Ampulle) zu mindestens 250 ml einer kompatiblen Infusionslösung zugesetzt und innerhalb von 2 bis 3 Stunden infundiert. Das Zuspritzen zu der Infusionslösung soll erst unmittelbar vor der Anwendung und unter aseptischen Kautelen erfolgen.

Als Basislösung sind reine Glukoselösungen (5 % bis 70 % Glukose) oder reine Kochsalzlösungen zu verwenden. Die Medikation kann auch zu einer Ringer-Lösung zugesetzt werden, wobei darauf zu achten ist, dass dies keine Ringer-Lactat-Lösung sein darf.

Die Medikation kann prinzipiell über einen peripher- oder zentralvenösen Zugang appliziert werden. Der Zugang hängt laut Elmadfa und Leitzmann 1990 sowie Semsroth 1994 entscheidend von der indizierten Dauer der parenteralen Ernährung, vom pH-Wert, von der Osmolarität sowie der chemischen Zusammensetzung der Infusionslösung ab. Bei der kurzfristigen Infusionstherapie wird der Katheter periphervenös gelegt. Soll die parenterale Nahrungsversorgung länger als drei Tage erfolgen, wird über einen zentralvenösen Zugang infundiert. Patienten werden eingeschlossen, die mindestens 5 Tage parenteral ernährt werden und zudem eine Zumischung der Medikation zu einer anderen Infusionslösung erfolgen muss, wird geraten, dass die Applikation der Medikation über einen zentralvenösen Zugang stattfindet. Die Entscheidung über die Art des Zugangs liegt jedoch beim Arzt.

Die Substitution der Medikation sollte allerdings nur über einen Katheter erfolgen, über den keine Katecholamine gegeben werden. Eine weitere Vermischung der Medikation mit anderen zu infundierenden Substanzen ist vor der Infusion zu vermeiden. In keinem Fall sollte die Medikation mit Reduktionsmitteln wie beispielsweise Vitamin C gemischt werden, da hier Interaktionen z.B. mit Natriumselenit möglich sind und eine Ausfällung von elementarem Selen nicht auszuschließen ist. Elementares Selen ist in wässrigem Medium nicht löslich und nicht bioverfügbar. Ist eine Gabe von reduzierenden Substanzen notwendig, so sollte diese generell räumlich und wenn möglich auch zeitlich getrennt von der Gabe der Medikation erfolgen.

### Ergebnisse der Untersuchung an 52 parenteral ernährten Patienten

In einer Untersuchung an 52 parenteral ernährten Patienten mit Blutungen, Polytrauma/Trauma und Tumoren im Vogtland-Klinikum Plauen GmbH wurde auf Basis der Routinemedikation und -supplementation der Spurenelementstatus der Patienten im Therapieverlauf bestimmt. Weitere Ein- bzw. Ausschlusskriterien wurden bei der Auswahl der Patienten nicht zugrunde gelegt.

Ziel war es zu ermitteln, ob die routinemäßig verabreichte Spurenelementsubstitution zu einer zufriedenstellenden Spurenelementversorgung führt. Dazu wurde täglich der Serum- und Vollblutspiegel der Spurenelemente Chrom, Eisen, Fluor, Iod, Kupfer, Mangan, Molybdän, Selen, Zink über mindestens drei, höchstens 18 Tage bestimmt.

Die Gehaltsbestimmung von Mangan, Chrom, Selen und Molybdän in Serum- und Vollblutproben erfolgte mittels Graphitrohr-Atomabsorptionsspektroskopie (Graphitrohr-AAS) nach dem Standardadditionsverfahren (Gerätetyp: Perkin Eimer Analyst 600).

Die Bestimmung von Jod und Fluor wurde nach dem Standardadditionsverfahren mit ionensensitiven Elektroden durchgeführt.

Die Patienten erhielten während der genannten Beobachtungsdauer zu verschiedenen Zeitpunkten und in verschiedenen Dosierungen die normale Routinesupplementierung mit den Präparaten Inzolen HK, Inzolen^{®} sine NaCL, selenase^{®} und Unizink^{®}. Durch diese Präparate wurden neben den Mengenelementen Chlorid, Kalium, Magnesium und Natrium die Spurenelemente Kobalt, Kupfer, Mangan, Selen und Zink verabreicht.

Der bisherigen Routine entsprechend, wurden die Inzolen^{®}-Gaben vom täglich bestimmten Serum-Kalium-Wert abhängig gemacht, für den der Normbereich angestrebt wurde. Die Zink- und Selengaben erfolgten aufgrund von Erfahrungswerten in bestimmten Erkrankungssituationen. Die oben genannte Bestimmung der weiteren Mengen- und Spurenelementspiegel waren lediglich für eine retrospektive Betrachtung des Spurenelementstatus vorgesehen und wurden nicht in den täglichen Entscheidungsprozess zur eventuellen Supplementation einbezogen.

Die verabreichte Menge an Inzolen HK, Inzolen^{®} sine NaCL, selenase^{®} und Unizink^{®} sowie die daraus resultierenden Mengen an Kupfer, Mangan, Selen und Zink können der Tabelle 3, Tabelle 4 und Tabelle 5 entnommen werden.

**Tabelle 3 Gabe von Unizink^{®}, Inzolen HK, Inzolen^{®} sine Na und selenase^{®}**

| Präparat | Anzahl der substituierten Patienten | Bereich in ml | Gabe gesamt im Mittel in ml | Gabe im Mittel in ml pro Tag |
|---|---|---|---|---|
| Inzolen HK | 20 | 2 - 80 | 141 | 19 |
| Inzolen^{®} sine Na | 41 | 40 - 1900 | 489 | 52 |
| Selenase^{®} | 38 | 20 - 260 | 99 | 11 |
| Unizink^{®} | 21 | 60 - 450 | 227 | 18 |

**Tabelle 4 Substituierte Gesamtmenge an Zink, Kupfer, Mangan und Selen durch die Präparate Unizink^{®}, Inzolen HK, Inzolen^{®} sine Na und selenase^{®}**

| Element | Anzahl der substituierten Patienten | Bereich | Mittelwert | Median | Einheit |
|---|---|---|---|---|---|
| Kupfer | 44 | 3,56 - 199,28 | 46,20 | 28,47 | mg |
| Mangan | 44 | 1,98 - 110,75 | 25,68 | 15,82 | mg |
| Selen | 38 | 1.000,00 - 13.000,00 | 4.934,00 | 4.500,00 | µg |
| Zink | 44 | 6,28 - 645,79 | 152,24 | 95,73 | Mg |

**Tabelle 5 Gabe pro Tag an Zink, Kupfer, Mangan und Selen durch die Präparate Unizink^{®}, Inzolen HK, Inzolen^{®} sine Na und selenase^{®}**

| Element | Anzahl der substituierten Patienten | Bereich | Mittelwert | Median | Einheit |
|---|---|---|---|---|---|
| Kupfer | 44 | 1,19 - 15,00 | 4,30 | 5,08 | mg |
| Mangan | 44 | 0,66 - 8,57 | 2,83 | 2,39 | mg |
| Selen | 38 | 91,00 - 1.000,00 | 574,00 | 608,00 | µg |
| Zink | 44 | 2,09 - 38,10 | 14,54 | 11,51 | mg |

**Folgende Referenzbereiche wurden bei der Auswertung zugrunde gelegt:**

| | Chrom in nmol/l | Eisen in µmol/l | Fluor in µmol/l | lod in µmol/l | Kupfer in µmol/l |
|---|---|---|---|---|---|
| Serum | 0-19,23 | w 10,74 - 25,96 m 14,33 - 30,08 | 0,26 - 1,05 | 0,32 - 0,63 | 9,44 - 26,75 |
| Vollblut | 0-96,16 | 7.520,82 - 10.027,76 | - | 0,26 - 0,61 | 11,02 - 19,51 |
| | | | | | |

| | Mangan in nmol/l | Molybdän in nmol/l | Selen vin µmol/l | Zink in µmol/l | |
|---|---|---|---|---|---|
| Serum | 5,46 - 54,61 | 0 - 10,42 | 1,28 - 1,71 | 9,18 - 19,88 | |
| Vollblut | 127,42 - 191,12 | 10,42 - 104,23 | 1,53-2,05 | 61,17 - 114,70 | |

Bei der statistischen Auswertung wurde zunächst berechnet, bei wie vielen Patienten (in Prozent) die Spurenelementspiegel am jeweiligen Tag unterhalb, innerhalb, bzw. oberhalb des Referenzbereiches lagen. Im zweiten Schritt wurde ermittelt, wie viel Prozent der Spurenelementspiegel der Patienten im gesamten Beobachtungszeitraum (3 - 18 Tage) unterhalb, innerhalb und oberhalb des Referenzbereiches lagen.

Zudem wurden die Verlaufskurven der Spurenelementspiegel im Serum und Vollblut eines jeden Patienten bewertet, wobei die Durchführung weiterer Behandlungen/Therapien (Op, CT, Begleitmedikation etc.) Berücksichtigung fand.

### Selen

Verlauf des Selenspiegels aller Patienten berechnet in % bezogen auf den Referenzbereich:

| Selen Serum | Tag | 1 | 2 | 3 | 4 | 5 | 6 | 7 | 8 | 9 | 10 | 11 | 12 | 13 | 14 | 15 | 16 | 17 | 18 | Ø |
|---|---|---|---|---|---|---|---|---|---|---|---|---|---|---|---|---|---|---|---|---|
| Unterhalb | % | 92 | 92 | 88 | 65 | 59 | 38 | 35 | 30 | 36 | 44 | 53 | 46 | 27 | 40 | 50 | 50 | 33 | 44 | 46 |
| Innerhalb | % | 2 | 8 | 8 | 27 | 24 | 35 | 30 | 26 | 36 | 39 | 24 | 8 | 27 | 20 | 10 | 10 | 33 | 33 | 20 |
| Oberhalb | % | 6 | 0 | 4 | 8 | 18 | 27 | 35 | 43 | 27 | 17 | 24 | 46 | 45 | 40 | 40 | 40 | 33 | 22 | 34 |

| Selen Vollblut | Tag | 1 | 2 | 3 | 4 | 5 | 6 | 7 | 8 | 9 | 10 | 11 | 12 | 13 | 14 | 15 | 16 | 17 | 18 | Ø |
|---|---|---|---|---|---|---|---|---|---|---|---|---|---|---|---|---|---|---|---|---|
| Unterhalb | % | 96 | 98 | 94 | 89 | 76 | 59 | 65 | 70 | 64 | 75 | 72 | 79 | 77 | 83 | 75 | 82 | 90 | 60 | 80 |
| Innerhalb | % | 4 | 2 | 4 | 11 | 15 | 37 | 35 | 26 | 36 | 20 | 28 | 21 | 8 | 0 | 17 | 9 | 0 | 30 | 15 |
| Oberhalb | % | 0 | 0 | 2 | 0 | 9 | 4 | 0 | 4 | 0 | 5 | 0 | 0 | 15 | 17 | 8 | 9 | 10 | 10 | 5 |

### Beobachtungen aus den Verlaufskurven:

Die meisten Patienten wiesen über den gesamten Beobachtungszeitraum einen Selenmangel auf, der durch erniedrigte Selenkonzentrationen sowohl im Serum als auch im Vollblut deutlich wurde.

Erniedrigte Selenkonzentrationen im Blut konnten besonders bei Patienten mit multiplen Operationen, Sepsis, Peritonitis, Infektionen gemessen werden. Wurde Selen in diesen Fällen in hoher Dosis (1000 µg/d) substituiert, so stiegen die Selenserumwerte unmittelbar an, sanken jedoch sofort wieder ab, wenn die Selenzufuhr endete.

Zu beobachten war zudem, dass die Selenserumwerte bis in den Referenzbereich stiegen, jedoch die Vollblutwerte nur knapp den Referenzbereich erreichten. Aus den Ergebnissen wird geschlossen, dass bei diesen Patienten eine langfristig hohe und kontinuierliche Selensubstitution mit 1000 µg/d erforderlich ist.

Die wenigen Patienten, bei denen die höhere Selenkonzentrationen im Serum auf eine Selenüberdosierung hindeutete, wiesen im Vollblut immer einen im Referenzbereich liegenden Selenspiegel auf.

Es konnte eine geringere Infektionsneigung bei einem höheren Selenspiegel als bei einem erniedrigten beobachtet werden.

### Zink

Verlauf des Zinkspiegels aller Patienten berechnet in % bezogen auf den Referenzbereich:

| Zink Serum | Tag | 1 | 2 | 3 | 4 | 5 | 6 | 7 | 8 | 9 | 10 | 11 | 12 | 13 | 14 | 15 | 16 | 17 | 18 | Ø |
|---|---|---|---|---|---|---|---|---|---|---|---|---|---|---|---|---|---|---|---|---|
| unterhalb | % | 76 | 85 | 78 | 86 | 80 | 68 | 63 | 50 | 43 | 33 | 16 | 20 | 7 | 8 | 8 | 9 | 10 | 10 | 38 |
| Innerhalb | % | 20 | 13 | 22 | 14 | 17 | 29 | 38 | 50 | 52 | 62 | 68 | 73 | 79 | 92 | 92 | 91 | 80 | 80 | 49 |
| oberhalb | % | 4 | 2 | 0 | 0 | 3 | 4 | 0 | 0 | 4 | 5 | 16 | 7 | 14 | 0 | 0 | 0 | 10 | 10 | 14 |

| Zink Vollblut | Tag | 1 | 2 | 3 | 4 | 5 | 6 | 7 | 8 | 9 | 10 | 11 | 12 | 13 | 14 | 15 | 16 | 17 | 18 | Ø |
|---|---|---|---|---|---|---|---|---|---|---|---|---|---|---|---|---|---|---|---|---|
| unterhalb | % | 6 | 18 | 12 | 14 | 17 | 7 | 8 | 4 | 0 | 0 | 0 | 7 | 7 | 0 | 0 | 0 | 0 | 0 | 5 |
| innerhalb | % | 84 | 75 | 80 | 81 | 77 | 89 | 83 | 88 | 91 | 95 | 89 | 87 | 86 | 100 | 92 | 91 | 90 | 80 | 88 |
| oberhalb | % | 10 | 8 | 8 | 5 | 6 | 4 | 8 | 8 | 9 | 5 | 11 | 7 | 7 | 0 | 8 | 9 | 10 | 20 | 7 |

### Beobachtungen aus den Verlaufskurven:

Die Bestimmung der Zinkspiegel im Serum sowie im Vollblut ergab, dass diese Spiegel größtenteils über den gesamten Verlauf im Mittel im Referenzbereich lagen und somit als normal anzusehen sind.

Auffallend war jedoch, dass ein niedriger Zinkwert im Serum keinen intrazellulären Zinkmangel bedeuten, aber auch ein hoher Wert im Serum keinen intrazellulären Überschuss anzeigen muss.

Bei Patienten, die einen normalen Zinkvollblutspiegel aufwiesen und denen Zink zugeführt wurde, konnte beobachtet werden, dass selbst bei hoher Zinkzufuhr der Zinkserumwert kaum beeinflusst wurde. Der Einfluss von Zinkgaben auf den Vollblutspiegel des Zinks war noch weniger erkennbar. Normale Vollblutwerte stellen also offenbar einen stabilen Zustand dar.

War der Zinkvollblutwert eines Patienten allerdings erniedrigt, so bedurfte es mindestens einer Zufuhr von ca. 30 bis 35 mg Zink/d, um den Zinkvollblutwert auf Dauer zu normalisieren. Auffällig war, dass Patienten mit Mehrfachoperationen bzw. großen Wundflächen wie z.B. nach Verkehrsunfällen sehr hohe Zinkmengen benötigten. Zur Vorbeugung einer Mangelsituation ist deshalb eine entsprechend hohe Zinksubstitution unbedingt notwendig.

### Literaturverzeichnis:

Elmadfa und Leitzmann C: Ernährung des Menschen 2. überarb. Auflage, Stuttgart: Eugen Ulmer Verlag, 1990: 385 - 392
Gramm H J, Kopf A, Eyrich K: Spurenelementsupplementierung im Rahmen langzeitiger parenteralen Ernährungstherapien Blätter, P, Gramm, H J (Hrsg.): Mineralstorfe und Spurenelemente in der Ernährung der Menschen Blackwell Wissenschaft, Berlin, 1992: 34 - 44 ISBN 3 - 89412 - 115 - 7
Semsroth M: Parenterale Ernährung. Benzer, H, Burchardi, H, Larsen, R, et al. (Hrsg.): Intensivmedizin, z. korr. Auflage, Springer, Berlin, 1994: 120 - 150
Deutsche Gesellschaft für Ernährung: Referenzwerte für die Nährstoffzufuhr/Deutsche Gesellschaft für Ernährung (DGE) (Konzeption und Entwicklung: Arbeitsgruppe: "Referenzwerte für die Nährstoffzufuhr") 1. Auflage, Frankfurt am Main: Umschau/Braus: 2000
AMA American Medical Association, Department of Foods and Nutrition. Guidelines for essential trace element preparations for parenteral use. JAMA 214 (1979) 2051-2054.
National Advisory Group On Standards And Practice Guidelines For Parenteral Nutrition: Safe practices for parenteral nutrition formulations. Journal of Enteral and Parenteral Nutrition 22, 2 (1998) 49-66.
Hackl JM: Leitfaden der parenteralen Ernährung. Zuckschwerdt Verlag München (1992).
Shenkin A: Micronutrients and antioxidants in home parenteral nutrition. Clinical Nutrition 20 (Supplement 2) (2001) 47-50.
Rükgauer M, Kruse-Jarres JD: Verteilung der Spurenelemente in den Fraktionen des Vollbluts. 20. Mengen- und Spurenelemente, (2000) 137-145.
Heseker H: Fluorid - Funktionen, Physiologie, Stoffwechsel, Empfehlungen und Versorgung in der Bundesrepublik Deutschland. Ernährungs-Umschau 46 (1999) 8, 305-308.
Versieck J, Cornelis R: Normal levels of trace elements in human blood plasma or serum. Anal. Chim. Acta 116 (1980) 217-254.

## Patentansprüche

1. Spurenelementzusammensetzung Für die Ernährung in Form eines Elektrolytkonzentrats, **dadurch gekennzeichnet, dass** eine Tagesdosis der Zusammensetzung 0,5 - 2 mg Selen und 10 mg -100 mg Zink als Spurenelemente enthält, und wobei Eisen als Spurenelemente nicht enthalten ist.

2. Zusammensetzung nach Anspruch 1, **dadurch gekennzeichnet, dass** eine Tagesdosis der Zusammensetzung 1-2 mg Selen und 30 mg-100 mg Zink als Spurenelemente enthält.

3. Zusammensetzung nach Anspruch 1 oder 2, **dadurch gekennzeichnet, dass** die Zusammensetzung als Infusionslösung vorliegt.

4. Zusammensetzung nach Anspruch 3, **dadurch gekennzeichnet, dass** die Infusionslösung in einer für die parenterale Verabreichung geeigneten Form vorliegt.

5. Zusammensetzung nach einem der Ansprüche 1 - 4, **dadurch gekennzeichnet, dass** sie als wässrige Lösung für Injektionszwecke vorliegt.

6. Zusammensetzung nach einem der Ansprüche 1 - 5, **dadurch gekennzeichnet, dass** weitere Spurenelemente vorliegen, die ausgewählt werden aus Chrom, Fluor, Jod, Kupfer, Mangan und Molybdän.

7. Zusammensetzung nach einem der Ansprüche 1 - 6, **gekennzeichnet**, dass die Zusammensetzung als 10 ml Infusionslösung formuliert ist.

8. Verabreichungseinheit einer Zusammensetzung nach einem der Ansprüche 1 - 7, **dadurch gekennzeichnet, dass** sie als wässrige Lösung in einer Ampulle vorliegt.

9. Verwendung von Selen und Zink zur Herstellung einer Zusammensetzung zur Ernährung von Intensivpatienten, insbesondere von Sepsis-Patienten, **dadurch gekennzeichnet, dass** die Tagesdosis an Selen in dem Bereich von 0,5 mg - 2 mg und die Tagesdosis an Zink in dem Bereich von 10 mg - 100 mg liegt und dass bei der Verabreichung auf das Spurenelement Eisen verzichtet wird.

10. Verwendung nach Anspruch 9, **dadurch gekennzeichnet, dass** die Tagesdosis an Selen mindestens 1 mg und die Tagesdosis an Zink mindestens 30 mg beträgt.

11. Verwendung nach Anspruch 10, **dadurch gekennzeichnet, dass** die Spurenelemente parenteral verabreicht werden.

12. Verwendung nach einem der Ansprüche 9-11, **dadurch gekennzeichnet, dass** weitere Spurenelemente ausgewählt aus Chrom, Fluor, Jod, Kupfer, Mangan und Molybdän verabreicht werden.

13. Verwendung nach einem der Ansprüche 10 - 12, **dadurch gekennzeichnet, dass** die Spurenelemente über einen Zeitraum von mindestens 3 Tage, vorzugsweise mindestens 5 Tage, verabreicht werden.

14. Spurenelementzusammerisetzung zur Verwendung in der Ernährung eines Intensivpatienten in der Form eines Elektrolytkonzentrats, **dadurch gekennzeichnet, dass** eine Tagesdosis der Zusammensetzung 0.5 - 2 mg Selen und 10 mg - 100 mg Zink als -Spurenelemente enthält, wobei Eisen als Spurenelement nicht enthalten ist, und wobei die Zusammensetzung als Infusionslösung vorliegt.

## Claims

1. Nutrition trace element composition in the form of an electrolyte concentrate, **characterised in that** one daily dose of the composition contains 0.5 - 2 mg selenium and 10 mg - 100 mg of zinc as trace elements, wherein iron is not contained as a trace element.

2. Composition according to Claim 1, **characterised in that** the composition contains 1 mg - 2 mg of selenium and 30 mg -100 of zinc as trace elements.

3. Composition according to Claim 1 or 2, **characterised in that** the composition exist as an infusion solution.

4. Composition according to Claim 3, **characterised in that** the infusion solution exists in a form suitable for parenteral administration.

5. Composition according to any of Claims 1 - 4, **characterised in that** it exists as an aqueous solution for injection purposes.

6. Composition according to any of Claims 1 - 5, **characterised in that** further trace elements are present, which are selected from chromium, fluorine, iodine, copper, manganese and molybdenum.

7. Composition according to any of Claims 1 - 6, **characterised in that** the composition is formulated as a 10 ml infusion solution.

8. Administration unit of a composition according to any of Claims 1 - 7, **characterised in that** it exists as an aqueous solution in an ampoule.

9. Use of selenium and zinc in the preparation of a composition for nutrition of intensive care patients, particularly for sepsis patients, **characterised in that** the daily dosage of selenium is in the range of 0.5 mg - 2 mg and the daily dosage of zinc is in the range of 10 mg -100 mg and during application no iron is to be administered as a trace element.

10. Use according to Claim 9, **characterised in that** the daily dosage of selenium amounts to at least 1 mg and the daily dosage of zinc amounts to at least 30 mg.

11. Use according to Claim 10, **characterised in that** the trace elements are to be administered parenterally.

12. Use according to any of Claims 9 - 11, **characterised in that** further trace elements, selected from chromium, fluorine, iodine, copper, manganese and molybdenum, are to be administered.

13. Use according to any of Claims 10 - 12, **characterised in that** the trace elements are to be administered over a time range of at least three days, preferably at least five days.

14. Nutrition trace element composition for use in nutrition of an intensive care patient in the form of an electrolyte concentrate, **characterised in that** a daily dosage of the composition contains 0.5 mg - 2 mg selenium and 10 mg - 100 mg zinc as trace elements, wherein iron is not included as a trace element, and wherein the composition is an infusion solution.

## Revendications

1. Composition d'oligoéléments pour la nutrition, sous la forme d'un concentré d'électrolyte, **caractérisée en ce qu'**une dose quotidienne de la composition contient 0,5 - 2 mg de sélénium et 10 mg - 100 mg de zinc en tant qu'oligoéléments, et **en ce que** le fer n'est pas présent en tant qu'oligoélément.

2. Composition selon la revendication 1, **caractérisée en ce qu'**une dose quotidienne de la composition contient 1 - 2 mg de sélénium et 30 mg - 100 mg de zinc en tant qu'oligoéléments.

3. Composition selon la revendication 1 ou 2, **caractérisée en ce que** la composition est disponible sous forme de solution pour perfusion.

4. Composition selon la revendication 3, **caractérisée en ce que** la solution pour perfusion est disponible sous une forme appropriée à une administration par voie parentérale.

5. Composition selon l'une quelconque des revendications 1 à 4, **caractérisée en ce qu'**elle est disponible sous forme d'une solution aqueuse à des fins d'injection.

6. Composition selon l'une quelconque des revendications 1 à 5, **caractérisée en ce que** d'autres oligoéléments sont présents, lesquels sont choisis parmi le chrome, le fluor, l'iode, le cuivre, le manganèse et le molybdène.

7. Composition selon l'une quelconque des revendications 1 à 6, **caractérisée en ce que** la composition est formulée sous forme de solution de 10 ml pour perfusion.

8. Unité d'administration d'une composition selon l'une quelconque des revendications 1 à 7, **caractérisée en ce qu'**elle est disponible sous forme d'une solution aqueuse dans une ampoule.

9. Utilisation de sélénium et zinc pour la fabrication d'une composition destinée à la nutrition de patients en soins intensifs, en particulier de patients atteints de septicémie, **caractérisée en ce que** la dose quotidienne de sélénium se situe dans la plage de 0,5 mg - 2 mg et la dose quotidienne de zinc se situe dans la plage de 10 mg - 100 mg, et **en ce que**, lors de l'administration, l'oligoélément fer n'est pas présent.

10. Utilisation selon la revendication 9, **caractérisée en ce que** la dose quotidienne de sélénium est de l'ordre de 1 mg au moins et la dose quotidienne de zinc est de l'ordre de 30 mg au moins.

11. Utilisation selon la revendication 10, **caractérisée en ce que** les oligoéléments sont administrés par voie parentérale.

12. Utilisation selon l'une quelconque des revendications 9 à 11, **caractérisée en ce que** d'autres oligoéléments, choisis parmi le chrome, le fluor, l'iode, le cuivre, le manganèse et le molybdène, sont administrés.

13. Utilisation selon l'une quelconque des revendications 10 à 12, **caractérisée en ce que** les oligoéléments sont administrés pendant une période de 3 jours au moins, de préférence au moins 5 jours.

14. Composition d'oligoéléments, destinée à être utilisée dans la nutrition d'un patient en soins intensifs, sous la forme d'un concentré d'électrolyte, **caractérisée en ce qu'**une dose quotidienne de la composition contient 0,5 - 2 mg de sélénium et 10 mg - 100 mg de zinc en tant qu'oligoéléments, et **en ce que** le fer n'est pas présent en tant qu'oligoélément, et **en ce que** la composition est disponible sous forme d'une solution pour perfusion.
